# EUROPEAN PATENT APPLICATION

(11) **EP 4 595 748 A1**
(43) Date of publication of application: **06.08.2025**
(21) Application number: 25162193.4
(22) Date of filing: 23.06.2017
(51) Int. Cl.: A01N 1/125, A61F 2/10, A61F 2/28, A61L 27/36, A61L 27/38, A61L 27/54

(54) **VIABLE LYOPHILIZED COMPOSITIONS DERIVED FROM HUMAN TISSUES AND METHODS OF MAKING THE SAME**

(30) Priority: 24.06.2016 US 201662354362 P
(62) Divisional of application: 17816341.6
(71) Applicant: Osiris Therapeutics, Inc., Columbia, MD 21046 (US)
(72) Inventor: SINCLAIR, Steven, Ellicott City, 21043 (US); DANILKOVITCH, Alla, Columbia, 21045 (US); SATHYAMOORTHY, Malathi, Ellicott City, 21043 (US); KUANG, Jin-Qiang, Glenelg, 21737 (US); DHALL, Sandeep, Eldridge, 21075 (US); MELCHIORRI, Anthony, Ellicott City, 21043 (US)
(74) Representative: Secerna LLP

(57) **Abstract**

Disclosed are methods of lyophilizing a tissue sample comprising obtaining a tissue sample, contacting the tissue sample with a lyoprotectant solution, freezing the tissue sample, performing a first drying step of the tissue sample after freezing, and performing a second drying step of the tissue sample after the first drying step. Disclosed are lyophilized tissues prepared using the disclosed methods of lyophilizing a tissue sample comprising obtaining a tissue sample, contacting the tissue sample with a lyoprotectant solution, freezing the tissue sample, performing a first drying step of the tissue sample after freezing, and performing a second drying step of the tissue sample after the first drying step. Disclosed are methods of treating a wound or tissue defect comprising administering a reconstituted lyophilized tissue to the wound or tissue defect.

## Description

### BACKGROUND

The purpose of tissue preservation is to retain components (extracellular matrix (ECM), growth factors and endogenous viable cells) of fresh tissue intact, while providing an extended shelf-life for tissues compared to fresh tissue. Current tissue preservation methods include refrigeration, dehydration, and cryopreservation. However, all three methods suffer from certain drawbacks. Refrigeration of fresh tissues maintains high cell viability for a short time, which leads to a short shelf-life (weeks) and limited availability. Dehydration of tissues provides an extended shelf-life (years) for the tissue matrix that is retained, but leads to tissue devitalization that negatively impacts tissue biological function. Cryopreservation can retain living tissue cells for an extended time (months to years), but the cost and effort required to maintain ultra-low temperatures (-40°C or below) across the entire supply chain limits utilization. Given these drawbacks to currently available tissue preservation methods, pursuit of superior compositions and methods of tissue preservation that can (1) retain tissue structure and living cells, (2) provide an extended shelf-life (months to years), and (3) not require ultra-low temperatures for the supply chain are warranted. Such compositions and methods would have applications for military use, as well as clinical/commercial use.

### BRIEF SUMMARY

Disclosed herein are tissue samples and methods of preparing the tissue samples that allow for improved tissue preservation.

Disclosed are methods of lyophilizing a tissue sample comprising obtaining a tissue sample, contacting the tissue sample with a lyoprotectant solution, freezing the tissue sample, performing a first drying step of the tissue sample after freezing, and performing a second drying step of the tissue sample after the first drying step.

Also disclosed are methods of preparing a tissue sample comprising obtaining a tissue sample, contacting the tissue sample with a lyoprotectant solution, freezing the tissue sample, performing a first drying step of the tissue sample after freezing, performing a second drying step of the tissue sample after the first drying step and further comprising a step of reconstituting the lyophilized tissue.

Disclosed are lyophilized tissues prepared using the disclosed methods of lyophilizing a tissue sample comprising obtaining a tissue sample, contacting the tissue sample with a lyoprotectant solution, freezing the tissue sample, performing a first drying step of the tissue sample after freezing, and performing a second drying step of the tissue sample after the first drying step.

Disclosed are methods of treating a wound or tissue defect comprising administering a reconstituted lyophilized tissue to the wound or tissue defect. In some aspects, the tissue previously lyophilized tissue was lyophilized by one or more of the methods disclosed herein.

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.
Figure 1 is a bar graph showing cell viability of lyophilized skin graft compositions after rehydration.
Figure 2 is a bar graph showing cell viability of lyophilized skin graft compositions after rehydration (n=2 donors).
Figures 3A and 3B show fluorescence images. A) Shows cell viability of lyophilized amnion compositions. Representative images are shown (10X magnification) for stromal and epithelial layers. B) Shows cell viability of lyophilized chorion compositions. Representative images for each group are shown.
Figure 4 shows representative images of cell viability for lyophilized amniotic membrane compositions dried in vials or mounted flat on plastic applicators and placed in trays.
Figure 5 shows the evaluation of cell viability persistence over 24 hours after hydration for lyophilized amniotic membrane compositions mounted on plastic applicators and dried in trays. Total number of viable cells may slightly decrease over 24 hours, which is typical of both fresh and cryopreserved amniotic membranes.
Figure 6 is a table showing the appearance of lyophilized amniotic membrane compositions before and after rehydration. Membranes were soaked in the same solution and then lyophilized with or without the same solution. All membranes were mounted on plastic applicators, and in some cases, the top plastic applicator was cut from a solid square to a frame shape.
Figures 7A, 7B, and 7C show images of large amniotic membrane compositions. A) Shows two samples measuring 5 cm x 5 cm were mounted on to plastic applicators with holes on each piece, soaked in a trehalose solution, and then lyophilized without solution. B) Shows separate 5 cm x 5 cm samples that was mounted on one piece of plastic with holes and covered with a "Frame" plastic applicator, then submerged in trehalose solution for lyophilization. C) Shows the membrane after rehydration.
Figure 8 shows lyophilization of an amniotic membrane composition within a breathable autoclave bag.
Figure 9 shows cell viability of lyophilized amniotic membrane compositions compared to fresh and cryopreserved amniotic membrane controls. Samples were prepared and mounted on plastic applicators with either a square top piece or a frame top piece.
Figure 10 shows a representative live/dead image of cell isolated from lyophilized composition.
Figure 11 shows anti-inflammatory and immunomodulatory activity of viable lyophilized amniotic membrane compositions.
Figures 12A and 12B show cell viability and angiogenic activity of a viable lyophilized amniotic membrane. A) Shows live/dead staining of a viable lyophilized amniotic membrane composition on the day it was removed from the lyophilizer (Day 0). B) Shows angiogenic activity of a separate lyophilized sample in response to hypoxia + TNF + LPS, which can be attributed to the viable cells within the composition.
Figures 13A and 13B show the lack of immunogenic response against lyophilized amniotic membrane compositions. A) Shows release of TNFα, a marker of immune cell activation, in positive control was not observed for negative controls or experimental groups. B) Shows release of IFNy, another marker of activated immune cells, was comparable to the negative controls for both experimental groups.
Figure 14 shows stability of a viable lyophilized amniotic membrane composition.
Figure 15 shows cell viability of lyophilized micronized chorionic membrane compositions. All samples were treated with the same solutions and lyophilized in the same manner. Each group was processed from the same starting material and represents samples taken in succession during a micronizing process. Images of Group 1 and 2 clearly show the micronized sheets of chorionic membrane with cells still embedded in the tissue.
Figure 16 shows the uptake of FITC-trehalose by chorionic stromal cells in suspension.
Figure 17 shows the uptake of trehalose by native placental cells present in fresh placental membrane tissues. Both epithelial cells in the amniotic membrane and stromal cells in the chorionic membrane are able to readily uptake trehalose.
Figure 18 shows a comparison of cell survival for placental cells in suspension vs. cells embedded in matrix.
Figure 19 shows the uptake of FITC-trehalose by chondrocytes embedded in bovine cartilage matrix.
Figure 20 shows the appearance of viable lyophilized micronized cartilage.
Figure 21 shows cell viability of lyophilized bovine cartilage graft compositions. Only micronized cartilage compositions retained cell viability (~50%).
Figure 22 shows cell viability of viable lyophilized bone graft. Green dots - viable cells. Red color - autofluorescence of bone matrix.
Figure 23 shows the stability of viable cells within viable lyophilized amniotic membranes. Membranes were stored at room temperature for 90 days after lyophilization, and cells were isolated enzymatically and stained to assess viability. Quantification of cell viability showed ~66-70% living cells.

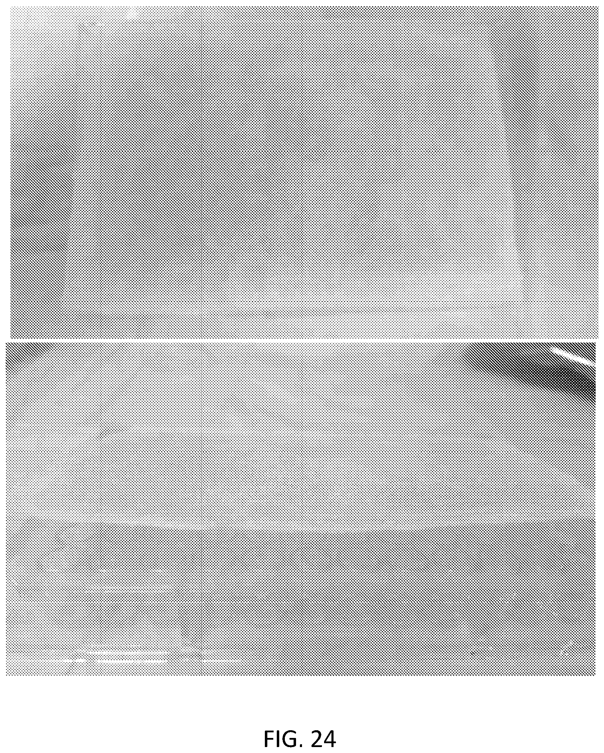

Figure 24 shows dry amniotic membrane overlaid on a medical-grade nylon mesh after lyophilization.
Figure 25 shows cell viability of viable lyophilized amniotic membranes using a lyoprotectant solution with trehalose only (10X magnification).
Figure 26 shows cell viability of viable lyophilized amniotic membranes using a lyoprotectants solution with trehalose and the antioxidant catechin. Cell viability is higher than with trehalose alone for the same lot. Images of epithelial layers and amnion stromal layer are included.
Figure 27 shows amniotic membrane isolated epithelial cells in tris buffer with trehalose and catechin.
Figure 28 shows amniotic membrane sheet in tris buffer with trehalose and catechin.
Figure 29 shows chorionic membrane minced in tris buffer with trehalose and catechin.
Figure 30 shows chorionic membrane minced in tris buffer with trehalose and EGCG.
Figure 31 shows live/dead stained fluorescent microscopic images of viable lyopreserved amniotic membrane (VLAM) post-rehydration in saline solution. Top images show viable and dead cells in epithelial (left) and stromal (right) layers in fresh amniotic membrane (AM). Bottom images show viable and dead cells in epithelial (left) and stromal (right) layers in VLAM post-rehydration.
Figure 32 shows cell viability of VLAM incubated in the lyopreservation solution for 60 or 105 minutes. Fresh AM was used as a control. The green line (70%) represents the acceptable cell viability criterion limit recommended by FDA for cellular therapies. Bars are mean % of cell viability +/- SD for 3 lots. Fresh is on far left, 60min incubation in the middle, 105 min incubation on far right.
Figure 33 shows the visual appearance of VLAM. The top row shows integrity of 3 lots of AM tissue (no cracks) after lyophilization. The bottom images show ease of sample detachment from the mesh when needed.
Figure 34 shows the cell viability of VLAM mounted on XN6080 mesh. The horizontal line (70%) represents the acceptable cell viability criterion limit recommended by FDA for cellular therapies. Bars are mean % of cell viability +/- SD for 3 samples tested for each lot.
Figure 35 shows the cell viability (%) of VCAM and VLAM (the 24hr. cycle prt2-MRM) using a new method of sample preparation without tissue digestion with trypsin. Bar graphs are mean % of cell viability+/- SD for 3 lots (3 samples from each lot). The horizontal line (70%) represents the acceptable cell viability criterion limit recommended by FDA for cellular therapies. Cryopreserved is column on the left. Lyophilized is column on the right.
Figure 36 shows the viable cell counts for VCAM and VLAM (the 24hr. cycle prt2-MRM) samples prepared with a modified method for sample preparation for cell viability assay without tissue digestion with trypsin. Bar graphs are mean total number of viable cells per 25 cm² +/- SD for 3 lots (3 samples from each lot). Cryopreserved is column on the left. Lyophilized is column on the right.
Figures 37and 37B show a graphical representation of the primary drying endpoint for the 24 hr lyophilization cycle for 25 (A) and 90 (B) AM unit load.
Figure 38 shows the position of temperature probes throughout AM unit stacks in the FTS Lyostar II.
Figure 39 shows the average temperature rate change at the top, middle and bottom positions for the VLAM stacks of all sizes during the 24 hr lyophilization cycle.
Figure 40 shows the temperature rate change during the freezing phase at the top, middle and bottom for the VLAM stacks of all sizes during the 24 hr lyophilization cycle. Top, middle and bottom probe temperatures were averaged for all VLAM stacks and all 3 shelves.
Figure 41 shows the temperature rate change during the heating step of the primary drying phase at the top, middle and bottom for the VLAM stacks of all sizes during the 24 hr lyophilization cycle. Top, middle and bottom probe temperatures were averaged for all VLAM stacks and all 3 shelves.
Figure 42 shows the average temperature rate change at the middle position for each size of the VLA stacks during the 24 hr lyophilization cycle.
Figure 43 shows the cell viability of viable cryopreserved amniotic membrane (VCAM) and VLAM (the 24hr. cycle prt2-MRM) after the 24 hr lyophilization cycle. Fresh AM was used as a control. Bar graphs are mean % of cell viability +/- SD for 3 lots (3 samples from each lot). The horizontal line (70%) represents the acceptable cell viability criterion limit recommended by FDA for cellular therapies.
Figure 44 shows the visual appearance of VLAM units with implemented pre-lyophilization treatment in the 0.045 M trehalose solution.
Figure 45 shows the cell viability of VLAM treated by incubation versus rinse with a 0.045 M trehalose solution. Fresh AM served as a control. Bar graphs are mean % of cell viability +/- SD for 4 lots (3 samples from each lot). The horizontal line (70%) represents the acceptable cell viability criterion limit recommended by FDA for cellular therapies.
Figure 46 shows the cell viability of VLAM units stored at -80°C for 97 hr prior to lyophilization. VLAM units lyophilized immediately after packaging served as a control. Bar graphs are mean % of cell viability +/- SD for 3 lots (3 samples from each lot). The horizontal line (70%) represents the acceptable cell viability criterion limit recommended by FDA for cellular therapies.

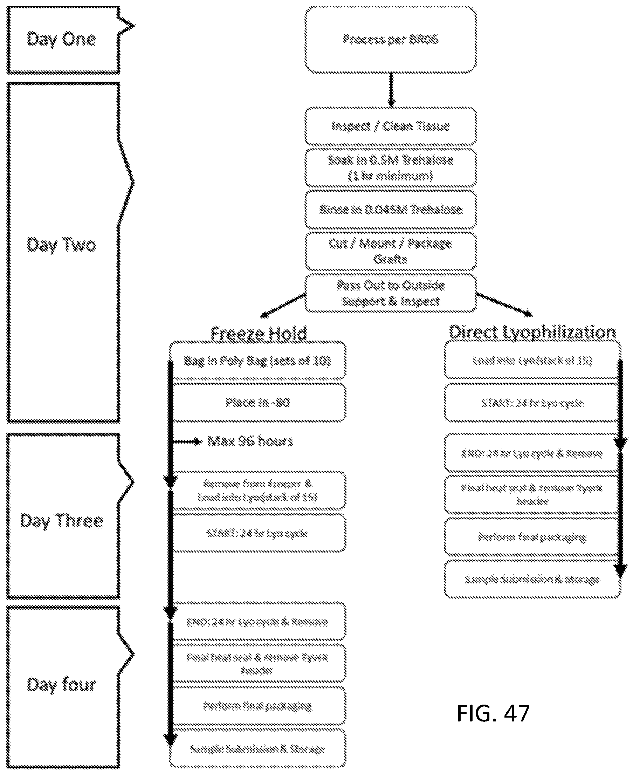

Figure 47 is a flow chart of steps for the VLAM manufacturing.
Figure 48 shows the cell viability of VLAM units with the 5h 20 min "lag time" post-packaging prior to at -80°C for 97 hr prior to placing into a lyophilizer. VCAM units served as a control. Bar graphs are mean % of cell viability +/- SD for 3 lots (3 samples from each lot). The horizontal line (70%) represents the acceptable cell viability criterion limit recommended by FDA for cellular therapies.
Figure 49 shows the cell viability of VLAM units exposed to 37°C for 77 hrs. 34 min. VCAM units tested after lyophilization served as a control. Bar graphs are mean % of cell viability +/- SD for 3 lots (3 samples from each lot). The horizontal line (70%) represents the acceptable cell viability criterion limit recommended by FDA for cellular therapies.
Figure 50 shows the cell viability of VLAM units exposed to 50°C for 92 hrs. 15 min. VCAM units tested after lyophilization served as a control. Bar graphs are mean % of cell viability +/- SD for XX lots (samples). The horizontal line (70%) represents the acceptable cell viability criterion limit recommended by FDA for cellular therapies.
Figures 51A, B, C, D, E, and F showH&E staining of (a) VLAM, (b) VCAM, and (c) fresh amniotic tissue and MT staining of (d) VLAM, (e) VCAM, and (f) fresh amniotic tissue
Figure 52 shows a visual presentation of wounds in mice after 1st and 6th applications of a control dressing (Tegaderm), VCAM, or VLAM.
Figure 53 shows a time course of wound area reduction after applications of control dressing, VCAM, or VLAM.
Figure 54 shows histological images of mouse wound tissue collected post-closure after VCAM and VLAM applications. H&E staining shows tissue structure and MT staining shows collagen deposition.
Figure 55 shows H&E staining of fresh amnion, VCAM, and VLAM after 6 months of storage in ambient conditions.

### DETAILED DESCRIPTION

The disclosed method and compositions may be understood more readily by reference to the following detailed description of particular embodiments and the Example included therein and to the Figures and their previous and following description.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Definitions

It must be noted that as used herein and in the appended claims, the singular forms "a ", "an", and "the" include plural reference unless the context clearly dictates otherwise. Thus, for example, reference to "a tissue sample" includes a plurality of such tissue samples, reference to "the tissue sample" is a reference to one or more tissue samples and equivalents thereof known to those skilled in the art, and so forth.

"Native cells" means cells that are native, resident, or endogenous to the tissue sample, i.e. cells that are not exogenously added to the tissue sample.

"Native factors" means factors that are native, resident, or endogenous to the tissue sample, i.e. factors that are not exogenously added to the tissue sample.

"Substantially free" means present in only a negligible amount or not present at all. For example, when a cell is abundant less than about 20% or less than about 10% or less than about 1% of the amount in an unprocessed sample.

"Substantial amount" of an element of the present invention, e.g. native factors, therapeutic factors, or selective depletion, means a value at least about 2% or at least 10% in comparison to an unprocessed, fresh tissue sample. A substantial amount can optionally be at least about 50%.

"Therapeutic cells" as used herein means viable cells native to a given tissue that have retained their native biological functions to dynamically respond to a local microenvironment, for example an injury site or wound. Examples of therapeutic cells include, but are not limited to, fibroblasts, epithelial cells, MSCs, and other tissue-specific cell types, such as osteoblasts or osteoclasts for bone, or CD34+ follicular cells of the skin epidermis, or chondrocytes of hyaline cartilage, or fibrochondrocytes of meniscus, or annulus fibrosus or nucleus pulposus cells of the intervertebral disc, or supportive cell types surrounding peripheral nerve.

"Therapeutic factors" means tissue-derived factors that promote wound healing or tissue regeneration. For example, placenta- or chorionic membrane- derived factors that promote wound healing or tissue regeneration. Examples include, but are not limited to IGFBP1, adiponectin, α2-macroglobulin, and bFGF. Other examples include, but are not limited to MMP-9 and TIMP1. Other therapeutic factors include, but are not limited to, TGF-beta 1, beta 2, or beta 3, HGF, VEGF, IGF-1, and BMPs.

"Stromal cells" refers to a mixed population of cells present (optionally in native proportions) composed of mesenchymal stem cells and fibroblasts natively found within the stromal layer of a given tissue type.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range¬ from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

As used herein, "kit" means a collection of at least two components constituting the kit. Together, the components constitute a functional unit for a given purpose. Individual member components may be physically packaged together or separately. For example, a kit comprising an instruction for using the kit may or may not physically include the instruction with other individual member components. Instead, the instruction can be supplied as a separate member component, either in a paper form or an electronic form which may be supplied on computer readable memory device or downloaded from an internet website, or as recorded presentation.

As used herein, "instruction(s)" means documents describing relevant materials or methodologies pertaining to a kit. These materials may include any combination of the following: background information, list of components and their availability information (purchase information, etc.), brief or detailed protocols for using the kit, trouble-shooting, references, technical support, and any other related documents. Instructions can be supplied with the kit or as a separate member component, either as a paper form or an electronic form which may be supplied on computer readable memory device or downloaded from an internet website, or as recorded presentation. Instructions can comprise one or multiple documents, and are meant to include future updates.

In various aspects, the subject of the herein disclosed methods is a vertebrate, *e.g.,* a mammal. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. A patient refers to a subject afflicted with a disease or disorder. The term "patient" includes human and veterinary subjects.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Publications cited herein and the material for which they are cited are hereby specifically incorporated by reference. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinence of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of"), meaning that each step is not intended to exclude, for example, other additives, components, integers or steps that are not listed in the step.

Those skilled in the art will recognize, or be able to ascertain using no more than routine experimentation, many equivalents to the specific embodiments of the method and compositions described herein. Such equivalents are intended to be encompassed by the following claims.

### B. Methods of Lyophilizing

Disclosed are methods of lyophilizing a tissue sample comprising obtaining a tissue sample, contacting the tissue sample with a lyoprotectant solution, freezing the tissue sample, performing a first drying step of the tissue sample after freezing, and performing a second drying step of the tissue sample after the first drying step.

Also disclosed are methods of preparing a tissue sample comprising obtaining a tissue sample, contacting the tissue sample with a lyoprotectant solution, freezing the tissue sample, performing a first drying step of the tissue sample after freezing, performing a second drying step of the tissue sample after the first drying step and further comprising a step of reconstituting the lyophilized tissue. Reconstituted tissue of the disclosed methods can comprise at least 70% viable cells. In some aspects, reconstituted tissue can comprise greater than 40%, 45%, 50%, 55%, 60%, 65%, 70%, 75%, 80%, 85%, 90%, 95%, or 99% viable cells. In some aspects, after reconstituting the lyophilized tissue, the tissue can then be cut to a desired size. Percent viability of cells after reconstitution is based on the percent of viable cells that were in the starting tissue sample prior to being lyophilized.

### 1. Obtaining a tissue sample

In some aspects, obtaining a tissue sample can be performed by those methods known in the art. The method of obtaining a tissue sample can depend on the type of tissue sample being obtained. For example, obtaining a placental tissue can occur at the time of childbirth. In some aspects, tissue samples can be obtained from a cadaver.

In some aspects, a tissue sample can be, but is not limited to, a placenta or portion of a placenta, skin, bone, or cartilage. In some aspects, a placenta or placental tissue can be amniotic tissue, chorionic tissue, umbilical cord tissue, or a combination thereof. In some aspects, cartilage can be articular, hyaline or fibrocartilage. An example of fibrocartilage can be meniscal tissue.

In some aspects, a tissue sample does not comprise cultured cells. For example, the cells present in the tissue sample would be considered native to the tissue sample and non-cultured if the native cells have not previously been removed from the tissue sample and plated, seeded, cultured or in any other way allowed to adhere to a plastic or protein surface for any amount of time. Cells that have been previously removed from the tissue sample and plated, seeded, cultured or in any other way allowed to adhere to a plastic or protein surface for any amount of time are referred to herein as "cultured cells".

In some aspects, a tissue sample can be cut to a desired size. Cutting a tissue sample to a desired size can occur prior to freezing the tissue sample (i.e. before or after contacting the tissue sample with a lyoprotectant solution). In some aspects, a tissue sample can be minced. Mincing a tissue sample can occur prior to freezing the tissue sample (i.e. before or after contacting the tissue sample with a lyoprotectant solution).

In some aspects, a tissue sample can be treated with an antibiotic. In some aspects, a tissue sample can be treated with an antibiotic prior to freezing (e.g. before or after contacting the tissue sample with a lyoprotectant solution).

### 2. Contacting the tissue sample with a lyoprotectant solution

In some aspects, contacting the tissue sample with a lyoprotectant solution can include a short or prolonged contact. For example, the tissue sample can be exposed or contacted to a lyoprotectant solution for 1, 2, 3, 4, 5, 10, 15, 20, 25, 30, 35, 40, 45, 50, 55, or 60 minutes. In some aspects, the tissue sample can be exposed or contacted to a lyoprotectant solution for 1, 2, 3, 4, 5, 6, 7, 8, 9, 10, 12, 14, 16, 18, 20, 22, or 24 hours. In some aspects, the tissue sample can be exposed or contacted to a lyoprotectant solution for 1, 2, 3, 4, 5, 6, 7, 14, 21 days. In some aspects, the tissue sample can be exposed or contacted to a lyoprotectant solution for 1, 2, 3, 4, 5, 6, 7, or 8 weeks.

In some aspects, contacting the tissue sample with a lyoprotectant solution can be the same as exposing the tissue sample to a lyoprotectant solution or soaking the tissue sample in a lyoprotectant solution.

As described here, a lyoprotectant solution comprises at least one lyoprotectant. In some aspects, a lyoprotectant solution can comprise trehalose. Other lyoprotectants can include but are not limited to polyhydroxy compounds such as sugars, polyalcohols, raffinose, and other non-reducing polysaccharides, and their derivatives.

In some aspects, the lyoprotectant solution can further comprise one or more antioxidants. In some aspects, the one or more antioxidants can be epigallocatechin gallate (EGCG) or catechin. In some aspects, an antioxidant can be ascorbic acid, L-carnosine, spermine, phloretine, α-tocopherol, β-carotene, conenzyme Q10, lutein, melatonin, butylated hydroxytoluene, γ-tocopherol, lutein, N-acetyl-L-cysteine, mitoquinone, hydroquinone, lipoic acid, glutathione, carotenoids, polyphenols, retinol, tocotrienol.

In some aspects, lyoprotectant solution can also comprise saline, DMSO, antibiotics, bulking agents, excipients, or a combination thereof. In some aspects, the lyoprotectant can comprise other reagents that can improve lyophilization performance.

The concentration of a lyoprotectant or antioxidants present in the lyoprotectant solution and the length of time for contacting the tissue sample with the lyoprotectant solution can be dependent on the type and size of the tissue sample. Based oon the teachings herein, one of skill in the art using routine methods would understand how to adjust the concentrations and contacting times.

In some aspects, contacting the tissue sample with a lyoprotectant solution can occur at temperatures between 0° and 39°C. In some aspects, contacting the tissue sample with a lyoprotectant solution can occur at 4°C.

### 3. Freezing the tissue sample

In some aspects, freezing the tissue sample can be performed at a temperature range of -80°C to -4°C. In some aspects, freezing the tissue sample can be performed at a temperature range of -70°C to -4°C. In some aspects, freezing the tissue sample can be performed at a temperature range of -50°C to -4°C.

In some aspects, the sample can be added for purposes of freezing the tissue, wherein the tissue can be added prior to achieving the final freezing temperature. In some aspects, the step of freezing the tissue sample can involve avoiding a flash freeze and instead providing a steady cooling to freezing temperatures. In such instances, the temperature can be decreased at a rate between 0.1 and 10°C/min. In such instances, the temperature can be decreased at a rate between 0.1 and 5°C/min. In some instances, flash freezing can cause formation of water crystals that can kill the tissue-resident cells and alter the structure of the tissue matrix. In such instances, a slower freeze can be used to avoid killing the tissue or native cells contained therein.

### 4. Performing a first drying step of the tissue sample after freezing

In some aspects, the first drying step of the tissue sample after freezing occurs between -45°C and -15°C. In some aspects, the first drying step of the tissue sample after freezing occurs between -45°C and -10°C. In some aspects, the first drying step of the tissue sample after freezing occurs between -45°C and -5°C. In some aspects, the first drying step of the tissue sample after freezing occurs between - 45°C and 0°C. In some aspects, the first drying step of the tissue sample after freezing occurs between -45°C and +15°C. In some aspects, the first drying step of the tissue sample after freezing occurs between -45°C and +10°C. In some aspects, the first drying step of the tissue sample after freezing occurs between -45°C and +5°C. In some aspects, the temperature of the first drying step can be the same as the freezing temperature. In some aspects, the temperature of the first drying step can be at least 1°, 5°, 10°, 15°, 20°, 25°, 30°, 35°, 40°, 45°, or 50°C higher than the temperature of the freezing step.

In some aspects, the first drying step of the tissue sample after freezing can be carried out for less than 10 hours. In some aspects, the first drying step of the tissue sample after freezing can be carried out for 10, 12, 14, 16, 18, 20, or 24 hours. In some aspects, the first drying step of the tissue sample after freezing can be carried out for 24, 48 or 72 hours.

### 5. Performing a second drying step of the tissue sample after the first drying step

In some aspects, the second drying step can be carried out at a temperature that is greater than the temperature of the freezing step. In some aspects, the second drying step can be carried out at a temperature that is greater than the temperature of the freezing step and the first drying step.

In some aspects, the temperature is increased between the first drying step and the second drying step. In such aspects, the temperature of the second drying step is higher than the temperature of the first drying step. In some aspects, wherein the temperature of the second drying step is higher than the first drying step, the rate of the temperature increase from the first drying step can be gradual or rapid. For example, the rate of temperature increase from the first drying step to the second drying step can be from 0.1 to 5°C/min. In some aspects, the rate of temperature increase from the first drying step to the second drying step can be 0.33 to 1°C/min.

In some aspects, the second drying step can occur at a temperature of no more than 39°C. In some aspects, the second drying step can occur at a temperature of no more than 45°C.

In some aspects, the second drying step can be carried out at two or more different temperatures. In some aspects, the at least two different temperatures can be at least 5°, 10°, 15°, 20°, 25°, 30°, 35°, 40°, 45°, or 50°C different from each other. For example, the second drying step can be carried out at 0° and then at 20°C. In some aspects, the second drying step can be conducted in at least two different temperatures, wherein each different temperature can each be maintained for 5 to 15 minutes each. In some aspects, the temperature can be ramped up from one temperature to the next, each of the intervening temperatures can be maintained for about 10 sec to 1 minute. Thus, although the second drying step can be carried out at two or more different temperatures, many temperatures can be involved in the second drying step as the tissue sample is exposed to all of the temperatures in between the at least two temperatures that are maintained for 5-15 minutes.

In some aspects, the second drying step can be conducted at more than two different temperatures. For example, the second drying step can be conducted at 0°, 20°, and 30°C. In some aspects, the second drying step can be conducted in at least three different temperatures, wherein each different temperature can be each maintained for 5 to 15 minutes each. As the temperature is ramped up from one temperature to the next, each of the intervening temperatures can be maintained for about 10 sec to 1 minute. Thus, although the second drying step can be carried out at three or more different temperatures, many temperatures can be involved in the second drying step as the tissue sample is exposed to all of the temperatures in between the at least two temperatures that are maintained for 5-15 minutes.

In some aspects, the second drying step is conducted for 12-144 hours. In some aspects, the second drying step is conducted for 12-48 hours. In some aspects, the second drying step is conducted for 12-72 hours. In some aspects, the second drying step is conducted for at least 12, 15, 12, 25, 0, 35, 40, 45, 50, 55, 60, 65, 70, 75, 80, 85, 90, 95, 100, 105, 110, 115, 120, 125, 130, 135, 140, or 144 hours.

In some aspects, after drying the lyophilized tissue, the tissue can be cut to a desired size or shape.

### C. Lyophilized Tissue

Disclosed are lyophilized tissues prepared using the methods disclosed herein.

Disclosed are lyophilized tissues prepared using the methods disclosed herein that are sealed inside a sterile package.

**In** some aspects, the lyophilized tissue disclosed herein can be stable for at least three weeks. In some aspects, the lyophilized tissue can be stable for at least three months. In some aspects, the lyophilized tissue can be stable for 1, 2, 3, 4, 5, 6, 7, 8, 9, 10, 11, 12, 24, 36, 48, or 60 months.

In some aspects, the lyophilized tissue disclosed herein can be reconstituted resulting in a reconstituted tissue. Lyophilized tissue can be reconstituted using standard techniques known in the art. In some aspects, reconstituting refers to rehydrating. Thus, the disclosed lyophilized tissues can be reconstituted or rehydrated using water, saline, a buffer such as, but not limited to phosphate buffered saline (PBS), in a solution comprising a stabilizing agent such as, but not limited to bovine serum albumin (BSA), Plasma-Lyte A or other clinically available electrolyte solutions, with human bodily fluids or a combination thereof. For example, lyophilized tissue can be applied directly to a wound or tissue injury on a subject and the subject's bodily fluids can reconstitute. In some aspects, a combination of bodily fluids and another known rehydrating solution can be used. Also, disclosed are reconstituted tissue prepared using the methods disclosed herein.

The reconstituted tissue derived from the methods disclosed herein can comprise native viable cells and native therapeutic factors. The reconstituted tissue can comprise at least 40%, 45%, 50%, 55%, 60%, 65%, 70%, 75%, 80%, 85%, 90%, 95%, 99% viable cells compared to the same tissue prior to lyophilization. The reconstituted tissue can comprise at least 40%, 45%, 50%, 55%, 60%, 65%, 70%, 75%, 80%, 85%, 90%, 95%, 99% viable native cells compared to the same tissue prior to lyophilization.

### 1. Chorionic membrane

**In** some aspects, reconstituted tissue can be reconstituted chorionic membrane. In some aspects, reconstituted chorionic membrane can comprise about 1,000 to about 240,000 cells/cm² or about 20,000 to about 60,000 cells/cm². In some aspects, reconstituted chorionic membrane can comprise 20,000 to about 200,000 cells/cm², with a cell viability of at least about 70%.

In some aspects, reconstituted chorionic membrane can comprise at least: about 7,400 or about 15,000 or about 23,217, or about 35,000, or about 40,000 or about 47,800 of stromal cells per cm² of the reconstituted chorionic membrane. Thus, reconstituted chorionic membrane can comprise about 5,000 to about 50,000 of stromal cells per cm² of the reconstituted chorionic membrane.

In some aspects, reconstituted chorionic membrane can comprise native chorionic cells wherein at least: about 40%, or about 50%, or about 60%, or about 70%, or about 74.3%, or about 83.4 or about 90%, or about 92.5% of the native chorionic cells are viable. Thus, reconstituted chorionic membrane can comprise native chorionic cells wherein about 40% to about 92.5% of the native chorionic cells are viable.

In some aspects, reconstituted chorionic membrane can have a thickness of about 20 µm to about 600 µm.

In some aspects, reconstituted chorionic membrane secretes less than about any of: 420 pg/mL, 350 pg/mL, or 280 pg/mL TNF-α into a tissue culture medium upon placing a 2 cm x 2 cm piece of the reconstituted chorionic membrane in a tissue culture medium and exposing the reconstituted chorionic membrane to a bacterial lipopolysaccharide for about 20 to about 24 hours.

In some aspects, reconstituted chorionic membrane can be associated with part or all of an amniotic membrane.

### 2. Amniotic membrane

In some aspects, reconstituted tissue can be reconstituted amniotic membrane.

In some aspects, reconstituted amniotic membrane can comprise an epithelial cell layer, wherein the approximate number of cells per cm² of the reconstituted amniotic membrane is about 10,000 to about 360,000 or about 40,000 to about 90,000.

In some aspects, reconstituted amniotic membrane can comprise a thick basement membrane (comprising one or more of Collagen Type I, Ill, IV, laminin, and fibronectin).

In some aspects, reconstituted amniotic membrane can comprise a stromal cell layer. In some aspects, the reconstituted amniotic membrane can comprise at least: about 2,000, or about 2,400, or about 4,000 or about 6,000, or about 8,000, or about 10,000, or about 10,585, or about 15,000 stromal cells per unit cm² of the amniotic membrane. In some aspects, the reconstituted amniotic membrane can comprise about 2,000 to about 15,000 of stromal cells per cm² of the amniotic membrane. In some aspects, the reconstituted amniotic membrane can comprise stromal cells wherein at least: about 40%, or about 50%, or about 60%, or about 70%, or about 74.3%, or about 83.4 or about 90%, or about 92.5% of the stromal cells are viable after reconstitution.

In some aspects, reconstituted amniotic membrane can comprise a thickness of about 20 to about 250 µm.

**In** some aspects, reconstituted amniotic membrane can comprise low immunogenicity. In some aspects, reconstituted amniotic membrane can comprise secretes less than about any of: 420 pg/mL, 350 pg/mL, or 280 pg/mL TNF-α into a tissue culture medium upon placing a 2 cm x 2 cm piece of the reconstituted amniotic membrane in a tissue culture medium and exposing the reconstituted amniotic membrane to a bacterial lipopolysaccharide for about 20 to about 24 hours.

In some aspects, reconstituted amniotic membrane can comprise a layer of amniotic epithelial cells.

In some aspects, reconstituted amniotic membrane can comprise native amniotic cells that include for example, epithelial cells or stromal cells. In some aspects, the amniotic stromal cells include amniotic fibroblasts and/or amniotic MSCs.

In some aspects, reconstituted amniotic membrane can provide an analgesic effect, reduce scarring, or both.

In some aspects, reconstituted amniotic membrane can comprise anti-inflammatory proteins such as IL-1Ra and IL-10, antibacterial proteins such as defensins and allantoin (bacteriolytic proteins), and angiogenic and mitogenic factors that promote re-epithelialization such as EGF, HGF, and VEGF.

In some aspects, reconstituted amniotic membrane can comprise cells that are positive for CD73, CD90, CD105, and CD166 and negative for CD45, CD34, and CD31. In some aspects, reconstituted amniotic membrane can comprise cells that express HLA-G, cells that express IDO and FAS ligand, which likely contribute to immune tolerance, cells with a capacity to differentiate into 1- Human Amniotic Epithelial Cells (hAECs), cells with a capacity to differentiate to neural, hepatocyte, and pancreatic cells, cells that expression of CD49d by hAMSCs distinguishes hAMSCs from hAECs, hAMSCs that are positive for the embryonic cytoplasmic marker Oct-4 that plays a role in maintaining pluripotency and self-renewal, and hAECs that are positive for SSEA-3, SSEA-4, TRA-1-60, TRA-1-81, and negative for SSEA-4 and non-tumorogenic.

In some aspects, reconstituted amniotic membrane can be associated with part or all of a chorionic membrane.

### 3. Cartilage

### i. Articular cartilage

In some aspects, cartilage can be articular cartilage tissue. Thus, in some aspects, reconstituted tissue can be reconstituted articular cartilage.

In some aspects, reconstituted articular cartilage can comprise TFG-β1, TGF-β3, BMP-7, bFGF, IGF-1.

In some aspects, reconstituted articular cartilage can comprise at least about 500 cells/mm², 600 cells/mm², 700 cells/mm², 800 cells/mm², 1200 cells/mm², or 1500 cells/mm².

In some aspects, reconstituted articular can comprise at least about 100 cells/mm² or 200 cells/mm² of viable chondrocytes. In some aspects, reconstituted articular cartilage comprises at least 50%, 60%, 70%, 80%, 90%, or 95% viable chondrocytes.

### ii. Meniscal Tissue

In some aspects, cartilage can be meniscal tissue. Thus, in some aspects, reconstituted tissue can be reconstituted meniscal tissue.

In some aspects, reconstituted meniscal tissue can comprise greater than 25%, 30%, 35%, 40%, 45%, 50%, 55%, 60%, 65%, 70, 75%, 80%, 85%, 90%, or 95% viable cells. In some aspects, reconstituted meniscal tissue can comprise greater than 25%, 30%, 35%, 40%, 45%, 50%, 55%, 60%, 65%, 70, 75%, 80%, 85%, 90%, or 95% viable native cells.

In some aspects, reconstituted meniscal tissue can be non-immunogenic. For example, reconstituted meniscal tissue can have depleted amounts of one or more types of functional immunogenic cells. An absence of immunogenic cells can be further confirmed if the reconstituted meniscal tissue does not produce > 100 pg/ml of TNF-alpha upon stimulation with a bacterial immunogen, such as LPS, within about 24 hours of culture. In some instances, >5% of cells present in the composition can be immune cells however the composition would be considered absent of immunogenic cells if <5% of the viable cells are immune cells.

In some aspects, reconstituted meniscal tissue can have one or more growth factors native to the meniscal tissue. The growth factors can be one or more of TGF-β1, TGF-b3, bFGF, PDGF-AB, PDGF-BB, IGF-1, HGF, BMP-7, EGF, CTGF, BMP-2, BMP-6, and VEGF.

In some aspects, reconstituted meniscal tissue can comprise at least one of the collagen layers of human meniscus.

In some aspects, reconstituted meniscal tissue can comprise viable, native mesenchymal stem cells.

### 4. Bone

**In** some aspects, reconstituted tissue can be reconstituted bone or a bone repair product. In some aspects, a bone repair product can comprise cancellous bone fragments and periosteum containing angiogenic growth factor(s).

The particular types and concentration of the growth factor(s) in the bone or a bone repair product can depend on the particular donor. In some aspects, the concentrations of each growth factor can independently be at least 1 pg/mL, such as at least 2 pg/mL, 5 pg/mL, 10 pg/mL, 30 pg/mL, 40 pg/mL, 50 pg/mL, 60 pg/mL, 70 pg/mL, 80 pg/mL, 90 pg/mL, 100 pg/mL, 200 pg/mL, 300 pg/mL, 400 pg/mL, 500 pg/mL, 600 pg/mL, 700 pg/mL, 800 pg/mL, 900 pg/mL, 1000 pg/mL, 2000 pg/mL, 3000 pg/mL, 4000 pg/mL, 5000 pg/mL, 6000 pg/mL, 7000 pg/mL, 8000 pg/mL, 9000 pg/mL, 10000 pg/mL, 20000 pg/mL, 30000 pg/mL, 40000 pg/mL, 50000 pg/mL or more and each will generally independently vary from or from about 1 pg/mL to 50000 pg/mL, such as 10 pg/mL to 10000 pg/mL or 50 pg/mL to 5000 pg/mL, such as from or from about 100 pg/mL to 1000 pg/mL, 100 pg/mL to 800 pg/mL, 100 pg/mL to 600 pg/mL, 100 pg/mL to 400 pg/mL, 100 pg/mL to 200 pg/mL, 200 pg/mL to 1000 pg/mL, 200 pg/mL to 800 pg/mL, 200 pg to 600 pg/mL, 200 pg/mL to 400 pg/mL, 400 pg/mL to 1000 pg/mL, 400 pg/mL to 800 pg/mL, 400 pg/mL to 600 pg/mL, 600 pg/mL to 1000 pg/mL, 600 pg/mL to 800 pg/mL or 800 pg/mL to 1000 pg/mL of BRP. The growth factors present in the bone or a bone repair product include, for example, VEGF, bFGF, PDGF, IGF-1, IGF-2, TGF-β1, BMP-2 and/or BMP-7, and each can be present in a concentration range as set forth above. As an example, BRP provided herein can contain VEGF and the concentration of VEGF can be at least 1 pg/mL, such as at least 2 pg/mL, 5 pg/mL, 10 pg/mL, 30 pg/mL, 40 pg/mL, 50 pg/mL, 60 pg/mL, 70 pg/mL, 80 pg/mL, 90 pg/mL, 100 pg/mL, 200 pg/mL, 300 pg/mL, 400 pg/mL, 500 pg/mL, 600 pg/mL, 700 pg/mL, 800 pg/mL, 900 pg/mL, 1000 pg/mL, 2000 pg/mL, 3000 pg/mL, 4000 pg/mL, 5000 pg/mL, 6000 pg/mL, 7000 pg/mL, 8000 pg/mL, 9000 pg/mL, 10000 pg/mL, 20000 pg/mL, 30000 pg/mL, 40000 pg/mL, 50000 pg/mL or more, and generally will vary from or from about 50 pg/mL to 5000 pg/mL, such as from or from about 100 pg/mL to 1000 pg/mL, 100 pg/mL to 800 pg/mL, 100 pg/mL to 600 pg/mL, 100 pg/mL to 400 pg/mL, 100 pg/mL to 200 pg/mL, 200 pg/mL to 1000 pg/mL, 200 pg/mL to 800 pg/mL, 200 pg to 600 pg/mL, 200 pg/mL to 400 pg/mL, 400 pg/mL to 1000 pg/mL, 400 pg/mL to 800 pg/mL, 400 pg/mL to 600 pg/mL, 600 pg/mL to 1000 pg/mL, 600 pg/mL to 800 pg/mL or 800 pg/mL to 1000 pg/mL of BRP. It is understood that these levels are just provided as examples, and that the exact levels can depend on the particular growth factor, the particular donor, the method used for protein extraction (e.g. lysis method), the method used to quantify protein levels and other factors within the level of the skilled artisan.

By virtue of the presence of biologically active growth factors provided by the periosteum and bone component, in some aspects the bone repair products provided herein can contain a greater concentration of a growth factor (e.g. angiogenic growth factors) than the concentration of the same growth factor in a corresponding product that does not contain periosteum (e.g. a product containing cancellous bone matrix only or cancellous/DBM only). In particular, bone repair product provided herein can contain a greater concentration of an angiogenic growth factor (e.g. VEGF, bFGF, PDGF, or IGF-1) than the concentration of the same growth factor in a corresponding product that does not contain periosteum. For example, bone repair product can contain a concentration of angiogenic growth factor (e.g. VEGF, bFGF, PDGF, or IGF-1) that is at least 0.1-fold, 0.5-fold, 1-fold, 1.5-fold, 2-fold, 2.5-fold, 3-fold, 3.5-fold, 4-fold, 4.5-fold, 5-fold, 6-fold, 7-fold, 8-fold, 9-fold, 10-fold or more greater than the concentration of the same angiogenic growth factor in a corresponding bone graft not containing periosteum. Any one or more, two or more, three or more, or four or more of VEGF, bFGF, PDGF and/or IGF-1 or other angiogenic growth factor can be present in the increased amount compared to a corresponding product that does not contain periosteum. As an example, bone repair product can contain VEGF in a concentration that is at least 0.1-fold, 0.5-fold, 1-fold, 1.5-fold, 2-fold, 2.5-fold, 3-fold, 3.5-fold, 4-fold, 4.5-fold, 5-fold, 6-fold, 7-fold, 8-fold, 9-fold, 10-fold or more greater than the concentration of the same growth factor in a corresponding bone graft not containing periosteum. It is understood that in such examples, the cancellous bone and DBM in the compared products are substantially the same, but the products differ in the periosteal component of the bone and DBM (e.g. lacks the periosteum). In such examples, the presence of growth factors can be assessed under substantially the same conditions. Due to the increased levels of angiogenic growth factors in BRP, BRP exhibits angiogenic activity to induce angiogenesis, which is not achieved by a corresponding bone graft prepared using the same procedure but not containing periosteum.

In some aspects, reconstituted reconstituted bone or a bone repair product can comprise greater than 25%, 30%, 35%, 40%, 45%, 50%, 55%, 60%, 65%, 70, 75%, 80%, 85%, 90%, or 95% viable cells. In some aspects, reconstituted reconstituted bone or a bone repair product can comprise greater than 25%, 30%, 35%, 40%, 45%, 50%, 55%, 60%, 65%, 70, 75%, 80%, 85%, 90%, or 95% viable native cells.

In some aspects, the bone repair product provided herein is not immunogenic. For example, the bone repair product can be substantially free of endothelial cells or hematopoietic cells and other immunogenic components.

### 5. Skin

In some aspects, reconstituted tissue can be reconstituted skin.

In some aspects, reconstituted skin can comprise greater than 25%, 30%, 35%, 40%, 45%, 50%, 55%, 60%, 65%, 70, 75%, 80%, 85%, 90%, or 95% viable cells. In some aspects, reconstituted skin can comprise greater than 25%, 30%, 35%, 40%, 45%, 50%, 55%, 60%, 65%, 70, 75%, 80%, 85%, 90%, or 95% viable native cells.

In some aspects, reconstituted skin can be non-immunogenic. For example, reconstituted skin tissue can have depleted amounts of one or more types of functional immunogenic cells. An absence of immunogenic cells can be further confirmed if the reconstituted skin tissue does not produce > 100 pg/ml of TNF-alpha upon stimulation with a bacterial immunogen, such as LPS, within about 24 hours of culture. In some instances, >5% of cells present in the composition can be immune cells however the composition would be considered absent of immunogenic cells if <5% of the viable cells are immune cells.

In some aspects, reconstituted skin can have one or more growth factors native to the skin. The growth factors can be one or more of TGF-β1, TGF-b3, bFGF, PDGF-AB, PDGF-BB, IGF-1, HGF, BMP-7, EGF, CTGF, BMP-2, BMP-6, and VEGF.

In some aspects, reconstituted skin can comprise viable, native epidermal cells, dermal fibroblasts, and CD34+ stem cells.

In some aspects, reconstituted skin can comprise anti-bacterial factors, such as but not limited to RNase 7.

### D. Methods of Treating

Disclosed are methods of treating a wound or tissue defect comprising administering a reconstituted lyophilized tissue to the wound or tissue defect. Disclosed are methods of treating a wound or tissue defect comprising administering one or more of the reconstituted lyophilized tissues disclosed herein to the wound or tissue defect. For example, a wound can be selected from the group consisting of a laceration, a scrape, an abrasion, a thermal or chemical burn, an incision, a puncture, a wound caused by a projectile, a chronic wound, an acute wound, an external wound, an internal wound, a congenital wound, an ulcer, and combinations thereof. In some aspects, a wound or tissue defect can be in connection with surgery. For example, a surgery can be selected from the group consisting of a tendon surgery, a ligament surgery, a bone surgery, a spine surgery, a laminectomy, a knee surgery, a shoulder surgery, a hand surgery, an elbow surgery, a toe surgery, a foot surgery, an ankle surgery, a laprascopic surgery, an endoscopic surgery, robotic surgery, an open abdominal surgery, or combinations thereof.

Methods of administering a previously lyophilized tissue to a wound or tissue defect are known in the art. For example, the previously lyophilized tissue can be placed on a wound or tissue defect or can be surgically implanted/attached onto a wound or tissue defect.

### E. Kits

In one aspect, disclosed are kits comprising a disclosed lyophilized tissue and one or more of: (a) water, saline, or a buffer such as, but not limited to phosphate buffered saline (PBS), in a solution comprising a stabilizing agent such as, but not limited to bovine serum albumin (BSA), Plasma-Lyte A or other clinically available electrolyte solutions, with human bodily fluids or a combination thereof; and (b) instructions for reconstituting lyophilized tissue.

In various aspects, the lyophilized tissue and other compositions described herein can be provided in a kit. The kit can also include combinations of the lyophilized tissue, lyophilization agents, water, saline, or a buffer such as, but not limited to phosphate buffered saline (PBS), in a solution comprising a stabilizing agent such as, but not limited to bovine serum albumin (BSA), Plasma-Lyte A or other clinically available electrolyte solutions, with human bodily fluids or a combination thereof described herein.

In various aspects, the informational material can be descriptive, instructional, marketing or other material that relates to the methods described herein and/or to the use of the lyophilized or reconstituted tissue for the methods described herein.

In various aspects, the composition of the kit can include other ingredients, such as a solvent or buffer, a stabilizer, a preservative, a fragrance or other cosmetic ingredient. In such aspects, the kit can include instructions for the lyophilized or reconstituted tissue and the other ingredients, or for using one or more compounds together with the other ingredients.

### Examples

### A. Example 1

The most prevalent tissue preservation methods include refrigeration, dehydration, and cryopreservation. Refrigeration of fresh tissues is usually performed by incubating tissues in a particular electrolyte medium (e.g. Phosphate buffered saline (PBS), Dulbecco's Minimal Essential Medium (DMEM)) along with other additives or preservatives that may delay cell death within tissue. Refrigeration can maintain high structural integrity of the tissue, such as preservation of the extracellular matrix (ECM) proteins and natural porosity of the tissue. However, refrigeration of fresh tissues can only maintain cell viability for a short period of time, from a few days up to a 3-4 weeks depending upon the tissue type. Due to this short shelf-life and requirement of refrigerators, storage of fresh tissues has very limited availability commercially.

Conventional dehydration of tissues to remove water content can be achieved using three methods: 1) placing tissue in a warm oven for some time to evaporate water from the tissue; 2) passing an inert gas (e.g. argon, nitrogen) over the tissue to evaporate water from the tissue; or 3) freeze-drying (a.k.a lyophilization) of tissue by first freezing the tissue and then subjecting the tissue to a very low pressure (<3000 mTorr) using vacuum, which leads to sublimation--water in the solid phase is converted directly into the vapor phase. Current methods of dehydration lead to a disruption in the structural integrity of the tissue and presence of air pockets, or vacuoles, within the tissue ECM. Furthermore, all current dehydration methods lead to a devitalization or loss of tissue viable cells. All current dehydrated or lyophilized products, therefore, do not contain viable cells and are unable to preserve the cells biological function within fresh tissue. The primary advantage of dehydrated products is the long shelf-life of these tissue products, often 2 to 5 years, without the need for special equipment.

Cryopreservation of tissues is typically performed by adding cryoprotectants (e.g. DMSO, glycerol, etc.) at different concentrations to solutions and submerging tissues in these solutions before freezing. Tissues can be frozen with these cryoprotectant solutions at a controlled rate to an ultra-low temperature (-40C or below). The ultimate goal of cryopreservation is to maintain the structural and cellular integrity of the fresh tissue, but allow for longer storage times at ultra-low temperatures (-40C or lower typically). Currently, the only preservation method that has the potential to retain high cell viability for long periods of time is cryopreservation. For maximum post-thaw cell viability, each tissue type may require a different type or concentration of cryoprotectant, a different freezing rate, and a different final storage temperature. The primary drawback to cryopreservation is the need to maintain ultra-low temperatures for packaged tissue across the entire supply chain, from storage, to shipment, to end-user storage just prior to use.

Given these drawbacks to currently available tissue preservation methods, pursuit of superior compositions and methods of tissue preservation that can (1) retain living therapeutic cells, (2) provide an extended shelf-life (months to years), and (3) not require ultra-low temperatures for the supply chain is warranted.

Some investigators have demonstrated alternative methods for preserving cell suspensions (i.e. cells fully isolated from native tissues) and retaining cell viability, including lyophilization. These lyophilization methods often include the addition of reagents, salts, or additives, sometimes referred to as lyoprotectants, that exhibit protective mechanisms on cells during the desiccation process. Common lyoprotectants include DMSO, methylcellulose, sucrose, trehalose, antioxidants, human or animal serum proteins, and cellular stress proteins. Additionally, methods for increasing the transport of lyoprotectants inside cells in suspension have also been investigated as a way of improving the viability of cells after lyophilization. These methods include electroporation, addition of reagents that enhance intracellular transport, genetic modification of cells to upregulate the expression of pores on cell membranes, and mechanical microfluidic devices that partially disrupt cell membrane integrity and may promote intracellular transport of lyoprotectants.

Importantly, all of these methods to promote transport of lyoprotectants into cells are only effective on freely isolated cells in suspension. Gene therapy, electroporation, and enhancing intracellular transport or not effective for cells embedded in a native, dense tissue matrix. Hence, all previous reports of preservation of cell viability using lyophilization have exclusively focused on preserving cells in suspension, either freshly isolated from native tissues or isolated and culture-expanded cells. Lyophilization of mammalian cell suspensions has been demonstrated for platelets, mesenchymal stem cells (MSC), hematopoietic stem cells, among others. However, there are only very few examples limited to a particular type of tissue when endogenous cells were retained after lyophilization. In none of these cases was cell viability or immunogenicity assessed. This indicates that cells in free suspension can respond to dehydration or lyophilization differently.

Given the therapeutic benefits of fresh tissue grafts, which contains intact ECM, endogenous growth factors, and living endogenous cells, it is critical to have preservation methods that will retain all beneficial components of fresh tissue. Described herein are living lyophilized human tissue-derived compositions, and methods for generating the same, that can survive lyophilization and retain viable therapeutic cells upon rehydration, as well as biological function similar to the fresh tissue. This invention enables one to remove the costly cold chain required of cryopreserved living tissue compositions and represents a substantial improvement to the state of the art.

### 1. Experimental Methods

For these experiments, all human tissues were received from eligible donors after obtaining written informed consent, and tissue regulations for receipt and disposition of tissues was strictly followed. For some cartilage and bone tissue studies, bovine material was purchased from a local butcher.

### i. Skin Composition Processing

Human split-thickness skin grafts, containing a full epidermal and partial-dermal layer were, were recovered and transported on wet ice in a transport medium containing RPMI, cefazolin, and gentamicin sulfate to the inventors within 48 hours of asystole (death). Skin graft was removed from transport medium and soaked in chilled RPMI until the time when pieces were cut and shaped. For skin graft studies, biopsies of skin 12 mm in diameter were cut.

### ii. Placental Tissue Composition Processing

For placental tissues, full-term human placentas following vaginal or caesarean-section births were recovered and transported on wet ice in a typical transport medium to the inventors within 36 hours of delivery. Placentas were washed with saline to remove blood and the umbilical cord was cut and processed separately. Amniotic membranes were manually separated from the chorionic membrane and then cut with scissors to remove. Chorionic membranes were treated with dispase, or optionally soaked in DMEM without dispase, to loosen the membrane from the choriodecidua and decidua, which contain maternal blood cells and several other cell types that are immunogenic and should be avoided if the composition will be used clinically. The trophoblast layer was mechanically separated from the chorionic membranes. Both amniotic and chorionic membranes were washed with saline and mechanically cleaned to remove residual blood from the membranes. Optionally, membranes can be treated with a solution of ACD-A to prevent any further blood clotting. Once cleaned, the membranes were either immediately processed or submerged in a DMEM solution, optionally containing vancomycin, gentamicin sulfate, and amphotericin B, and incubated overnight at 37C. A sample of each fresh membrane was taken to perform cell viability testing as a positive control.

Both amniotic and chorionic membranes were then mounted onto nitrocellulose paper to facilitate cutting membranes into uniformly sized sheets, ranging in size from 1 x 1 cm to 8 x 12 cm. In some cases, membranes were minced or homogenized to create microscopic sheets of placental tissue prior to treating with solutions for lyophilization. Chorionic membranes were finely minced over 10-20 minutes using a curved stainless steel blade, and in some cases were further homogenized using a 7 mm or 12 mm probe attached to a motorized tissue homogenizer (PolyTron).

Additionally, placental tissue slurries comprised of umbilical cord tissue and amniotic membrane were also prepared by aggressive blending and homogenization. These slurries contained only minimal number of living cells or did not contain viable cells at all, but were used as additional human tissue-derived matrix for seeding cells in suspension or mixing with minced or homogenized matrix described above.

### iii. Cartilage Tissue Composition Processing

Articular cartilage grafts, either human or bovine, was isolated from the knee joint, sliced into 1-1.5mm thick pieces with varying surface areas. Some cartilage grafts were also porated using a 1 mm biopsy punch. Cartilage graft was also homogenized (PolyTron) into a cartilage slurry while on ice to maintain a cool temperature.

### iv. Bone Tissue Composition Processing

Cancellous bovine bone was cut into small pieces using a band-saw or air-powered sagittal saw and then fed through a mill to generate bone particles. These particles were then passed through a series of sieves to separate bone particles into different size ranges. Fat and any blood tissue components were mechanically separated from bone particles where possible.

### v. Tissue Treatments

Whole placental membranes or pre-cut membrane sheets were treated with varying reagents and solutions to promote cell survival and maintenance of tissue structure and integrity during lyophilization. In some cases, tissues were treated with solutions by submerging and incubating for some time before lyophilization. At which point, in some instances, tissues were then removed from such solutions just before lyophilization and submerged in a separate lyophilization solution. In other instances, the soaking solution and lyophilization solution were the same. Additionally, some studies including a soaking solution, but then tissue pieces were transported to drying containers without any lyophilization solution. Soaking times in all studies varied from 5 minutes to 4 hours.

Soaking solutions tested herein include typical cryopreservation solutions, such as an electrolyte solution (PlasmaLyte or 0.9% saline) with 5-10% DMSO and 2.5-5% human serum albumin, and alternative solutions such as 9-18% w/v trehalose (0.25M to 0.5M) in Saline with or without 0.1% w/v protamine. Trehalose has been previously shown to provide dessication protection for cells in suspension, but until now has not been applied to intact tissue pieces or micro-sheets or cells that still retain a pericellular matrix. Protamine is a positively-charge small protein that is known to promote intracellular transport mechanism of DNA or RNA. Tissue soaking was performed at room temperature or 37C, with or without shaking/agitation.

Lyophilization solutions tested herein include, in some instances, the same cryopreservation solutions above, as well as 100% fetal bovine serum, 25% human serum albumin, and combinations of the above cryopreservation solutions and human serum albumin.

### vi. Tissue Drying Containers and Configurations

Prior to beginning lyophilization, tissue pieces were placed into various containers to determine optimal methods and configurations. Standard cryovials, 5 cc glass vials, shallow plastic-trays, and plastic Petri dishes were used as containers for tissue during drying. Screw-tops and rubber stoppers for the cryovials and glass vials, respectively, were loosened prior to loading into the lyophilizer to allow vapor flow out of the container. Typically, 1 to 2 ml of lyophilization solution was added to cryovials or glass vials. Plastic trays were tested without any covers or with plastic covers that were cauterized to the trays at a few points, leaving much of the perimeter without a seal to permit air flow during lyophilization. Additionally, in some cases, amniotic and chorionic membranes were first placed between two plastic applicators, with one applicator having holes in the plastic, before placing into plastic trays for drying. Amniotic and chorionic membranes were also soaked prior to mounting on the plastic applicators, or soaked while mounted within the plastic applicators. For plastic trays, 2 to 5 ml of lyophilization solution was added, or enough to fully submerge the membranes.

Additionally, some membrane compositions were packaged as above and then placed into a breathable autoclave bag and sealed. The autoclave bag will permit air flow during lyophilization, but can keep a sample sterile, in the case that a sample must be transported from a sterile cleanroom environment to an outside room with a lyophilizer that is not a cleanroom.

### vii. Freeze-Drying Parameters

For these studies, an industrial-scale freeze-dryer (MillRock) was used to lyophilize samples. In the studies disclosed here, the same lyophilization cycle parameters were used for each study. In brief, samples were loaded onto shelves at room temperature (20-25°C), shelves were cooled to about -30°C to -70C at 0.1 to 5°C/min, and held for 30 to 240 min at the freezing temperature. A vacuum was applied once the temperature reached -30C and the chamber pressure was reduced to 100 mTorr. Primary drying was achieved by lowering the vacuum pressure to 20 mTorr and raising the shelf temperature to between -50C and -10°C at a rate of 0.1 to 5°C/min, and holding this temperature for at least 60 minutes. Primary drying was immediately followed by a three-phase secondary drying. In the first phase, the vacuum pressure was held at 20 mTorr and the shelf temperature was raised to -10 to 15°C at a rate of 0.1 to 5°C/min and held for 60 to 400 minutes. In the second phase, the vacuum pressure was held at 20 mTorr and the shelf temperature was raised to between 0 and 20°C at a rate of 0.1 to 5°C/min and held for 60 to 400 minutes. In the third and final phase of secondary drying, the vacuum pressure was held at 20 mTorr and the shelf temperature was raised to between 20 and 50°C and held for 1000 to 3000 minutes to remove additional residual moisture.

### viii. Evaluation of Appearance and Structure Pre- and Post-Rehydration

After the lyophilization cycle was complete, samples were removed and either immediately tested, or sealed in mangar pouches and stored at room temperature (20-25C) prior to experiments, including storage stability studies. Samples were evaluated for their dry appearance, absence of bubbling, orientation within drying containers after lyophilization, and cracking. To test mechanical integrity, amniotic and chorionic membranes mounted on plastic applicators were bent and flexed harshly prior to rehydration.

Tissues were rehydrated with water or saline solution for at least 2 minutes. After rehydration, the appearance of the tissue compositions, elasticity of membranes, color, and thickness were examined and compared to cryopreserved and fresh controls for each composition.

### ix. Measuring Cell Viability

The cell viability of fresh, cryopreserved, and lyophilized samples was measured using two different techniques. For the first technique, tissue samples were stained with LIVE/DEAD Cytotoxicity Kit (Life Technologies) to evaluate cell viability within compositions. For skin samples, cell viability of fluorescent images was quantified using an automated method with ImageJ software (NIH). For quantification of cell viability for amniotic and chorionic membranes a second technique was applied whereby cells were isolated by enzymatic digestion and then stained with Trypan Blue and counted using a hemacytometer. Viability of samples was measured immediately after hydration, and also post-hydration and after overnight culture at 37°C, 5% CO₂, to confirm cell viability persists over time after hydration. Cell viability was also tested for samples that were stored at room temperature for days and weeks after lyophilization.

### x. Isolation of Cells from Compositions

To isolate cells from lyophilized compositions or fresh or cryopreserved control samples, membranes were treated with a combination of trypsin and collagenase, for amniotic membranes, or collagenase only, for chorionic membranes, for 15-60 minutes, or until tissue was no longer visible to the eye. The resulting suspension was filtered to separate cells, then cells were washed, and could be plated for culture or tested for viability or functionality.

### xi. Evaluation of Anti-inflammatory and Immunomodulatory Properties

In addition to cell viability of compositions, the biological activity of lyophilized compositions in vitro were also tested. Fresh and cryopreserved placental tissues are known to possess anti-inflammatory and immunomodulatory activities. Using a well-known immunomodulatory assay, cryopreserved amniotic membrane was directly compared to a lyophilized amniotic membrane composition, as follows:
1. Thawed 1 vial of human peripheral blood mononuclear cells (PBMCs) for 2 ± min in a 37° C water bath.
2. Add 10 ml (each) of PBMC medium to a 15 ml conical tube.
3. Transfer thawed PBMCs from vial to tube with media.
4. Rinse each empty tube with the media/PMBC mixture to wash out any remaining cells, and replace media back in 15 ml conical tube.
5. Centrifuge at 1350 RPM for 6 min and remove supernatant.
6. Add 10 ml of medium to cell pellet to resuspend and achieve a concentration of ~1 x 10⁶ PBMC/ml.
7. Count cells.
8. Pipette 1000 µl of unstimulated PBMCs into wells (Negative Control).
9. Stimulate the remaining 8 ml PBMC solution by add 8 µl of anti-CD3 antibody and 8 µl of anti-CD28 antibody, for a final concentration of 10 ng/ml each.
10. Pipette 1000 ul of stimulated PBMCs into wells (Positive Control).
11. For cryospreserved amniotic membrane, placed two 3x4 cm samples (total of 24 cm2) into each well.
12. For lyophilized amniotic membrane compositions, place one 5x5 cm lyophilized sample (total of 25 cm2) into each well.
13. Add 1000 ul of activated PBMC solution to each well for experimental samples.
14. Incubate plates at 37o C, 5% CO2 for 48 hrs and collect supernatants.
15. Measure secreted tumor necrosis factor alpha (TNFα) and interferon gamma (IFNγ) in supernatants using ELISA.

### xii. Evaluation of Angiogenic response under hypoxic conditions

In the case of chronic wounds and acute soft tissue repair, cells at the wound/injury site are under hypoxic conditions and may also be exposed to high levels of pro-inflammatory cytokines or bacterial antigens. Fresh and cryopreserved amniotic membranes are known to respond to such harsh hypoxic conditions in vitro by secreting angiogenic factors like vascular endothelial growth factor (VEGF), whereas as dehydrated amniotic membrane without living cells does not respond in this manner. To simulate this harsh wound/injury environment and evaluate the responsiveness of lyophilized living tissue compositions, a previously reported assay was used to compare cryopreserved vs. living lyophilized amniotic membrane compositions, as follows:
1. Prepared culture media: 10% FBS in DMEM + 2% antibiotic/antimycotic + 50ug/mL gentamicin sulfate
2. Prepared stimulation media:
   a. Added 2µL of 100µg/mL TNF-α stock dilution to 20mL cell culture medium
   b. Added 20µL of 100µg/mL lipopolysaccharide (LPS) stock dilution to the cell culture medium with TNF-α.
3. Thawed multiple 3x4cm pieces of cryopreserved amniotic membrane.
4. Rehydrated multiple 5x5cm living lyophilized amnion compositions prepared with different methods.
5. Add tissue samples (24 to 25 cm2 total) to wells of 12-well plates.
6. Add 2 mL of stimulation medium to each of sample well to the stimulation plate. Add 2 mL of stimulation medium to empty wells as a control.
7. Add 2 mL of culture medium without TNF-α and without LPS to each sample well of the baseline plate. Add 2 mL of culture medium to an empty wells as control.
8. Place stimulation and baseline plates in hypoxic (O2 ~ 2%) and normal (O2 ~ 21%) conditions, respectively, for 96 hours.
9. Collect supernatant and tissue pieces separately. Lyse tissue samples to extract VEGF and measure levels of VEGF in supernatant and tissue extracts separately using ELISA.

### xiii. Evaluation of immunogenicity of viable lyophilized compositions

To demonstrate the absence of immunogenic factors and cells within viable lyophilized placental compositions, a previously published immunogenicity assay was used. Briefly, rehydrated lyophilized compositions and thawed cryopreserved controls were incubated at 37C for 24 hours in the presence of human PBMCs, and the secretion of TNFα was measured in the supernatant and compared to unstimulated PBMC (negative control) and PBMCs stimulated with another PBMCs derived from an independent donor (positive control).

### 2. Results

### i. Compositions of Viable Lyophilized Skin Grafts

Split-thickness skin graft biopsies (12 mm diameter) from n = 3 donors were cut and treated with varying soaking solutions and lyophilization solutions, then placed in cryovials and lyophilized. For one donor, the soaking and lyophilization groups are shown in Table 1.

**Table 1. Treatment Solutions for**

| Group | Treatment Solution | Treatment Time (min) | Lyophilizer Solution |
|---|---|---|---|
| 1 | 10% DMSO | 15 | 100% FBS |
| 2 | 100% FBS | 15 | 100% FBS |
| 3 | 10% Trehalose | 15 | 100% FBS |
| 4 | 1.6 mg/ml Protamine + 6.6% Trehalose | 15 | 100% FBS |
| 5 | 10% Trehalose | 15 | 10% Trehalose |
| 6 | 1.6 mg/ml Protamine + 6.6% Trehalose | 15 | 1.6 mg/ml Protamine + 6.6% Trehalose |

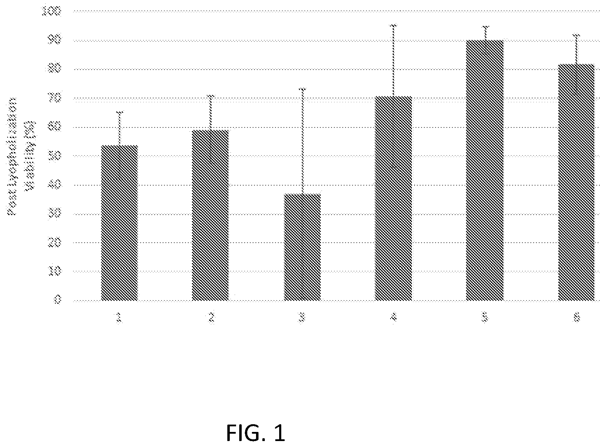

After lyophilization, skin samples from all groups appeared intact, without discoloration or cracking. The epidermal and dermal layers were still well connected. After rehydration, the handling properties of lyophilized skin was identical to fresh and cryopreserved skin. Qualitative analysis of live/dead staining indicated that Group 5 and Group 6 had better overall cell viability (~80-95%) compared to all other groups, where the lyophilization solution was 100% fetal bovine serum. Group 5 and Group 6 did not appear to lead to differences in cell viability, as shown in Figure 1.

In a separate experiment, skin biopsies from n = 2 donors were cut and divided into three groups: 1) soaking with 10% trehalose + 0.1% protamine for 90 min., then lyophilized in a solution with a final concentration of 12.5% human serum albumin (HSA), 5% trehalose, and 0.05% protamine; 2) the same soaking solution as Group 1 for 90 min., followed by lyophilization in 25% HSA; and 3) no soaking solution, then lyophilized in 25% HSA.

After lyophilization, skin samples for all groups had similar appearance and colors, without any separation of the epidermal and dermal layers or fracturing during lyophilization. After rehydration, skin samples for all groups exhibited handling properties identical to fresh or cryopreserved skin graft.

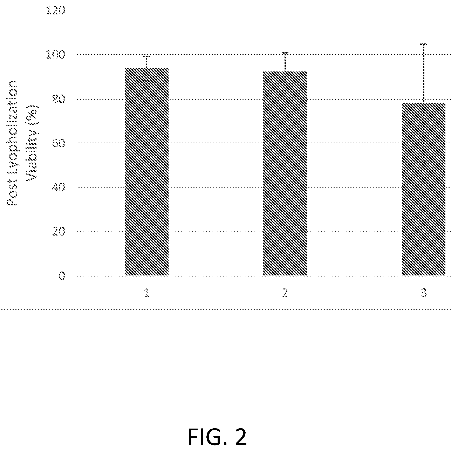

As shown in Figure 2, cell viability was substantially better for groups treated with trehalose + protamine (Group 1 and Group 2), showing cell viability of 90-100%. Lyophilization of skin in the presence of 25% HSA was superior for these donors compared to lyophilization in the presence of 100% FBS (Group 3).

For comparison, the typical viability of fresh skin graft within 5 days of tissue recovery is around 85-100%. Cryopreservation methods of the majority of skin allografts lead to <50% cell viability, but newer methods can retain viability above 70% and closer to 90% like fresh skin graft tissue. The lyophilized compositions derived from skin graft reported above retain cell viability at levels equivalent to fresh tissue.

### ii. Compositions of Viable Lyophilized Amniotic and Chorionic Membranes

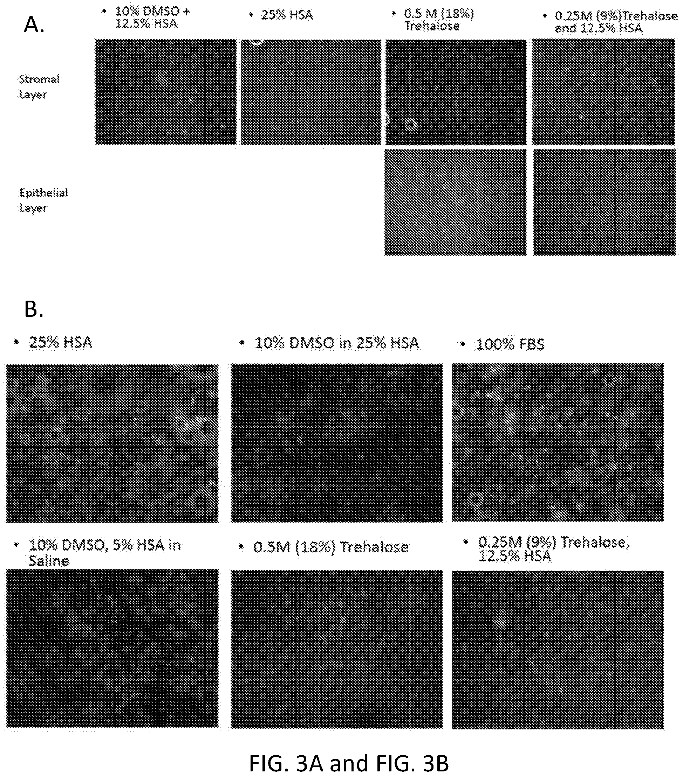

In the first study, amniotic and chorionic membranes from the same donated placenta were cut into 1 cm x 1cm pieces and treated with solutions that acted as the soaking solution and lyophilization solution. Samples were placed into 5 cc glass vials and covered with 1 ml of solution. In this configuration, both membrane types had a tendency to fold over in the vials, which leads to a less desirable appearance. The cell viability of these lyophilized placental composition was better for groups treated with trehalose, as shown in Figure 3A and 3B.

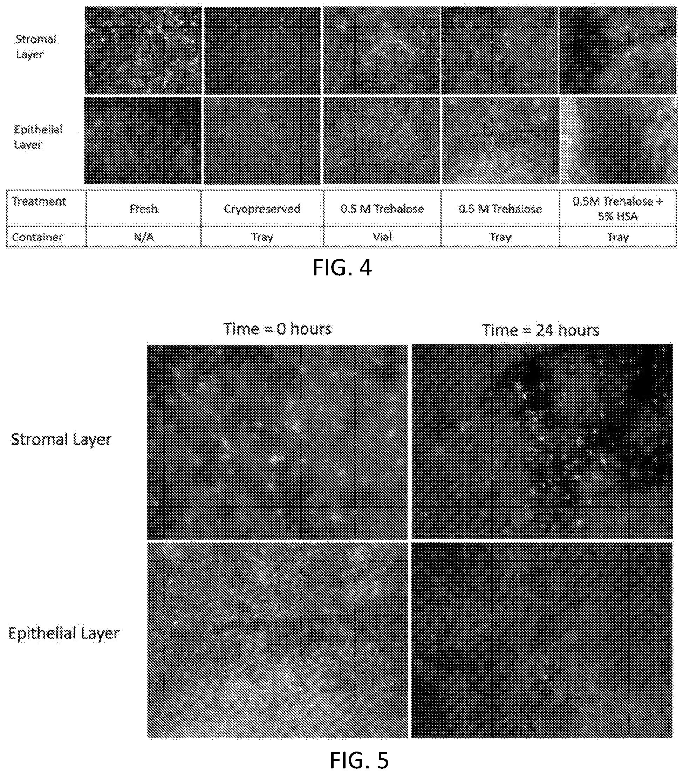

In a second study, larger sizes of lyophilized amniotic membrane compositions were made and the drying configuration and persistence of cell viability within compositions was investigated. Additionally, fresh amniotic membrane and cryopreserved amniotic membrane were used as positive controls for cell viability testing. Larger 3 cm x 4 cm amniotic membranes were prepared either moved to glass vials or mounted onto plastic applicators. The plastic applicators consist of a bottom plastic piece with holes and bottom plastic piece without holes that serve to contain the membranes and keep the membranes flat. Amniotic membranes in glass vials were submerged in 0.5M trehalose solution prior to lyophilization, and amniotic membranes mounted onto plastic applicators were placed in shallow plastic trays and submerged with 0.5M Trehalose or 0.5M Trehalose with 5% HSA. As shown in Figure 4, the cell viability of lyophilized amniotic membrane compositions was higher for membranes on plastic applicators compared to vials, likely because of the spread, even configuration of the membrane during drying. Lyophilization of these compositions led to post-hydration viability equivalent to fresh amniotic membrane. The presence of HSA in the lyophilization solution appeared to lessen viability for this donor.

As a follow-up of this study, the same amniotic membrane composition soaked with 0.5M Trehalose and then mounted on plastic applicators and dried in a tray was incubated at 37C, 5% CO2 in typical cell culture medium containing DMEM with 10% FBS for 24 hours. After 24 hours, the tissue was again stained to assess the cell viability 24 hours post-hydration. As shown in Figure 5, cell viability persists at 24 hours post-hydration, indicating that cell viability truly is maintained over time after hydration.

In a third study, the presence or absence of lyophilization solution was investigated, along with further investigation of the drying configuration by modifying the design of the plastic applicators for mounting the membrane. The appearance of lyophilized compositions and resistance to shattering or fragmentation.

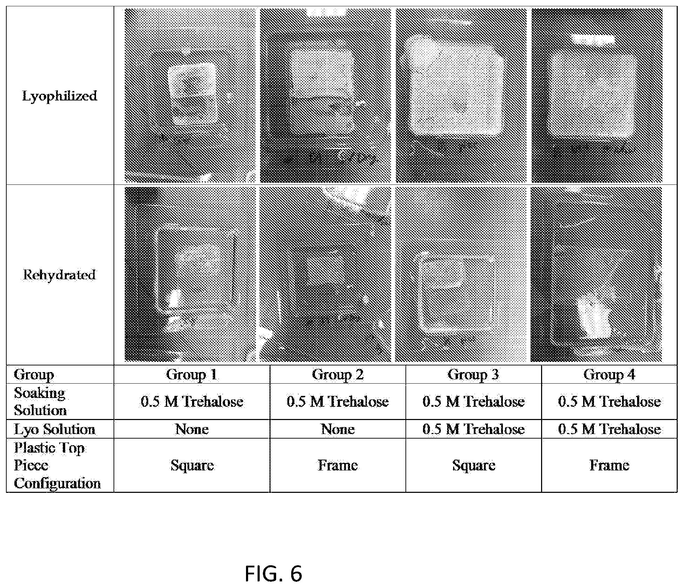

As shown in Figure 6, the appearance of compositions after lyophilization is different when lyophilization solution is absent compared to present (tissue was submerged). The trehalose solution does form a typical "cake" after lyophilization, but when the solution is absent during lyophilization, only residual solutes remain on the plastic applicator or adsorbed onto the membrane compositions. For some samples of Group 1 or Group 3, prior to rehydration while membranes were still mounted on plastic applicators, an operator grasped both edges of the configuration and harshly flexed the plastic to test for fracturing of the membrane. There was no fracturing, indicating the membranes remain stable within this drying configuration and the integrity of the membranes is preserved.

For 3 cm x 4 cm pieces, there was no evident fractures during the drying process, for either "Square" or "Frame" plastic applicator configurations. However, for larger 5cm x 5 cm pieces that were dried with a "Frame" plastic top applicator, some significant bending and tearing of the membrane occurred during lyophilization. Furthermore, for large 5 cm x 5 cm membrane compositions that were placed between two plastic applicators that both had holes with a "Square" configuration, some fracturing of membranes did occur during the lyophilization process. This indicates the configuration of the membranes and design of the mounting material is important to reducing fractures during drying.

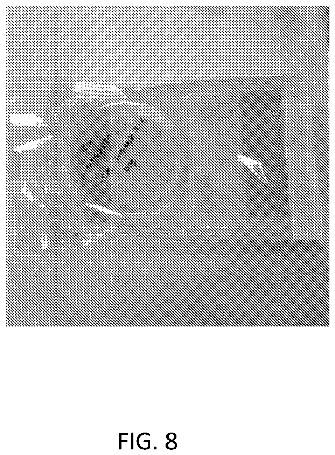

One additional lyophilization configuration that was tested was to enclose amniotic membrane compositions within a breathable autoclave bag. Given that many lyophilizers cannot be operated in a Class 7 or Class 8 clean room space, keeping clinical-grade product aseptic during transport from a cleanroom to a lyophilizer is paramount. Autoclave bags are used widely to store metal instruments for sterilization cycles and to be passed into a cleanroom. As shown in Figure 8, an amniotic membrane composition pre-soaked in a trehalose solution was then lyophilized without any solution and was successfully dried within the breathable autoclave bag.

After rehydration, all compositions have the same appearance, color, and handling properties as fresh amniotic membranes. Operators familiar with the handling of amniotic membranes could not differentiate thawed cryopreserved amniotic membranes from rehydrated lyophilized membrane compositions.

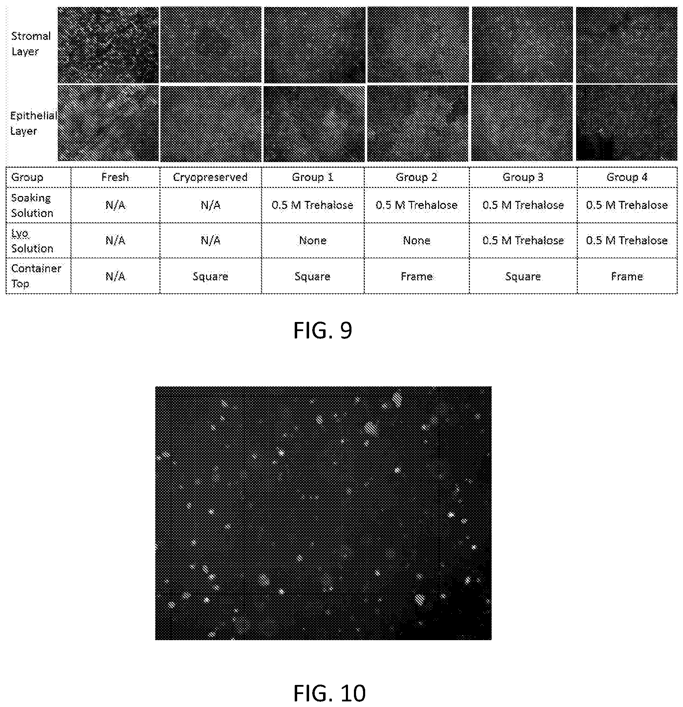

After evaluating appearance and handling properties, the cell viability of some 3 cm x 4 cm units were evaluated. As shown in Figure 9, groups frozen in the presence of solution (3, 4) appeared to have equivalent viability to groups frozen in the absence of solution (1,2). Groups dried with a "Square" top appeared to have higher cell viability that Groups dried with a "Frame" top.

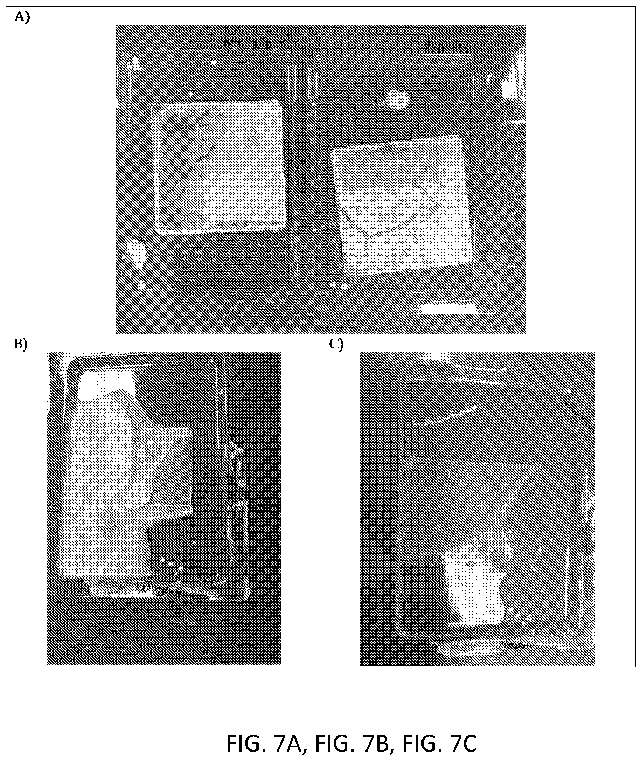

Cells present in lyophilized amniotic membrane compositions were isolated following rehydration and enzymatic digestion and then examined under the microscope and stained to evaluate cell viability of recovered tissues. The isolation protocol was as follows:
1. Rehydrate a 2x3cm amnion sample (Group 1 in Figure 7 above).
2. Place piece into well of a 6-well plate. Add 2 mL of 600 units/mL collagenase and 2 mL of 0.25% Trypsin-EDTA.
3. Incubate for 40min, observing how tissue looks under the microscope every 10 min.
4. After incubation period, transfer contents of well into gentleMACS (Miltenyl Biotec) C-tube.
5.Place C-tube into gentleMACS machine and run mouse spleen program 4.
6.Add 2 mL of 100% FBS to C-tube to neutralize the digestion solution.
7.Filter through 70 µm filter into 50mL tube.
8.Wash filter with 2mL of PBS.
9.Transfer contents from the 50 mL tube into 15 mL tube.
10.Centrifuge tube for 7 min at 2000 rpm.
11.Resuspend pellet in 1 mL of PBS.
12.Transfer content into microcentrifuge tubes, centrifuge for 3 min at 4000rpm, discard supernatants, then resuspend in 200 µL of live/dead stain.
13. Incubate in live/dead stain for ~15 min, then take images using the fluorescent microscope.

After isolation and staining, the cell viability was assessed. A representative image of isolated cells stained with live/dead are shown in Figure 10. The number of live and dead cells for this sample was counted using ImageJ software for three separate fields of view. Table 2 contains counting results, showing an average viability of cells isolated from one lyophilized amniotic membrane composition to be 78.9%.

**Table 2. Quantitation of cell viability for live/dead images of isolated cells.**

| | Live | Dead | Total | % Viability |
|---|---|---|---|---|
| Field of View 1 | 75 | 25 | 100 | 75 |
| Field of View 2 | 84 | 14 | 98 | 85.7 |
| Field of View 3 | 51 | 16 | 67 | 76.1 |
| Average | | | | 78.9 |

An additional 2cm x 3cm from Group 2 (Figure 9 above) was rehydrated and cells were isolated in a similar manner, then viability was counted using Trypan Blue and a hemacytometer. Table 3 contains cell counting data from two different operators, showing an average cell viability of 89% for this sample.

**Table 3. Quantitation of Cell viability of lyophilized amniotic membrane compositions using Trypan Blue and hemacytometer.**

| | **Live Cells** | **Dead Cells** | **Viability** |
|---|---|---|---|
| **Operator 1 Count** | 86 | 14 | 86% |
| **Operator 2 Count** | 168 | 15 | 92% |

### iii. Cells within Compositions of Viable Lyophilized Amniotic Membrane

### Possess Biological Activity

Given that cells remain viable in these lyophilized membrane compositions, the biological activity and functionality of those cells was investigated. Fresh placental membranes are known to have anti-inflammatory, immunomodulatory, and angiogenic activity, which can be linked back to the viable cells within fresh tissue. Some cryopreserved amniotic membranes, where high cell viability is retained, retain these biological functions. Therefore, the biological activity of viable lyophilized placental membrane compositions was tested.

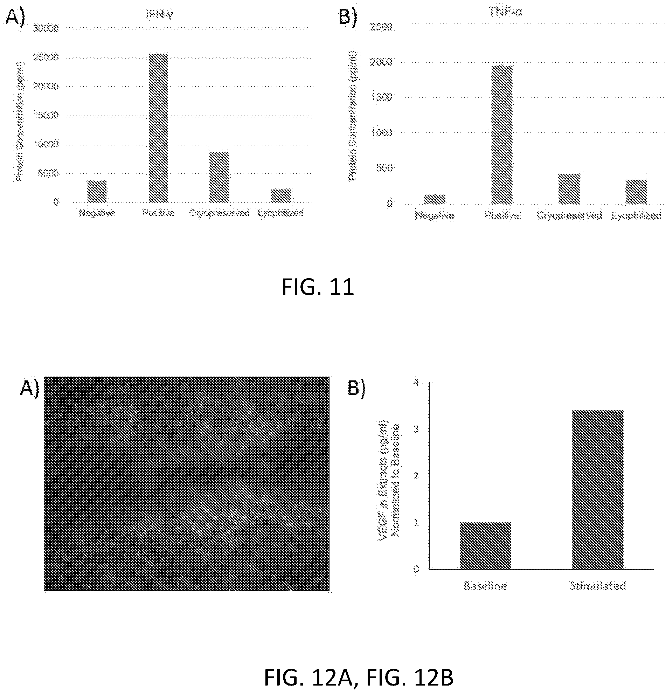

First, the anti-inflammatory and immunomodulatory function was tested using a previously published assay where PBMC are stimulated and incubated in the presence of membrane. The release of pro-inflammatory cytokines by stimulated PBMCs should be suppressed in the presence of an immunomodulatory composition. In this study, a cryopreserved amniotic membrane was compared to a viable lyophilized amniotic membrane composition and the secreted levels of TNFα and IFNγ were measured. Figure 11 shows the high anti-inflammatory and immunomodulatory activity of one viable lyophilized amniotic membrane composition, which showed higher activity than the cryopreserved control. This lyophilized sample was stored at room temperature for 9 days prior to hydration and use in this assay, indicating a prolonged stability of function.

In a second assay, the angiogenic activity of viable lyophilized amniotic membrane compositions within an in vitro model of a chronic wound/acute soft tissue injury was evaluated. Cryopreserved amniotic membranes are known to release higher amounts of VEGF into the supernatant or tissue matrix in response to the combination of hypoxia, TNFa (inflammation), and LPS (bacterial infection). Viable lyophilized amniotic membranes, which were stored at room temperature for 9 days prior to hydration and use in this assay, did respond to this in vitro model of a chronic wound/acute soft tissue injury by secreting 3.4 times more VEGF than the baseline case (Figure 12). Viable lyophilized amniotic compositions possess an angiogenic activity similar to fresh amniotic membranes.

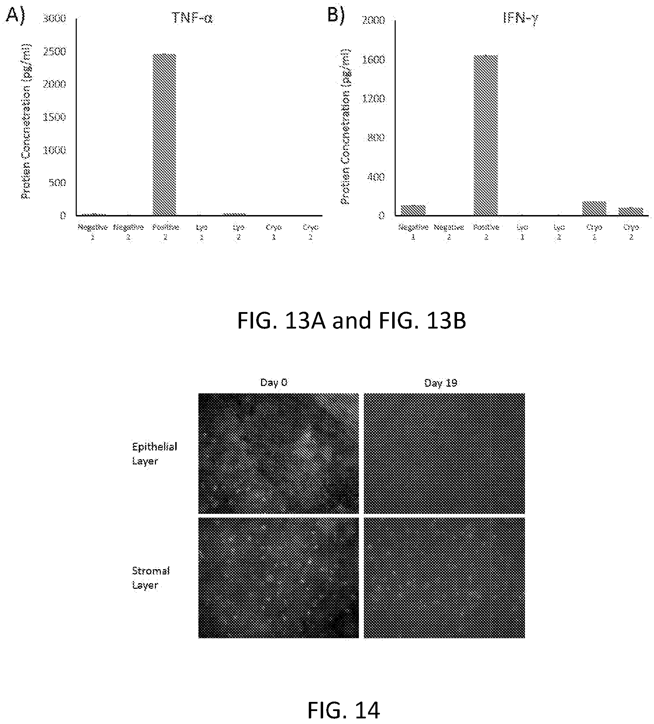

In a third assay, the immunogenicity of viable lyophilized amniotic membrane compositions was evaluated and compared to a cryopreserved amniotic membrane control. In this assay, lyophilized compositions or cryopreserved controls were incubated in the presence of human PBMCs and the levels of TNFα and IFNγ were measured. As a positive control, human PBMC from one donor were incubated in the presence of human PBMCs from a different donor, which will elicit an immune response characterized by increased secretion of TNFα and IFNγ. Hence, if lyophilized compositions or cryopreserved amniotic membrane controls contain immunogenic cells types, the levels of secreted TNFα and IFNγ should be increased. Conversely, if the lyophilized compositions contain negligible numbers of immunogenic cell types, the levels of both cytokines should be low. As shown in Figure 13, lyophilized compositions lacked an immunogenic response in this assay, and exhibited a lower response that the cryopreserved controls. This result, in combination with Figures 11 and 12, indicates that the lyophilized amniotic membrane compositions retain high levels of therapeutic cells and low levels of immunogenic cell types naturally present in placental tissues. The lyophilization methods reported herein can selectively deplete immunogenic cell types, while preserving therapeutic cell types.

### iv. Storage Stability of Viable Lyophilized Amniotic Membranes

The room temperature storage stability of viable amniotic membranes at room temperature was also investigated. Viable lyophilized amniotic membrane samples from two lots were stored at room temperature in sealed containers and protected from light. Samples stored for 14 days and 19 days were stained to evaluate cell viability, and found to still retain high cell viability, especially the amnion epithelial cells. Figure 14 shows a comparison of cell viability at Day 0 and Day 19 for one lot.

### v. Compositions of Minced and Micronized Viable Lyophilized Chorionic Membranes

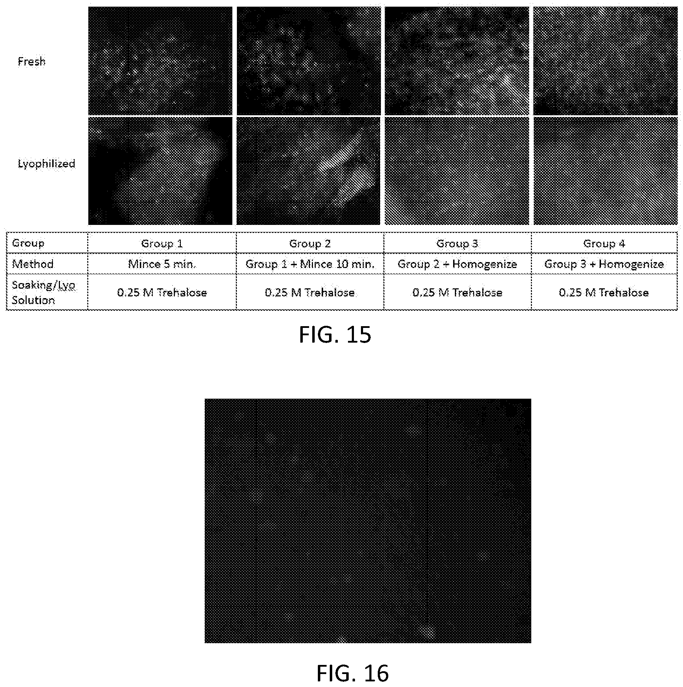

To further explore different embodiments of living lyophilized chorionic compositions, chorionic membranes were minced or micronized to create flowable chorionic dispersions with cells still embedded in the native placental matrix. In one study, chorionic membranes were successively minced and then homogenized, and samples of micronized membranes were taken over the course of processing. As shown in Figure 15, mincing and homogenizing did not impact the viability of the fresh tissue. Samples of membranes at four stages of the process were soaked in 0.5M trehalose solution for 4 hours at room temperature, then combined 1 to 1 with a devitalized placental tissue slurry previously prepared, and lyophilized. The devitalized tissue slurry was added to provide additional matrix and growth factors native to placental tissue. Viability of all 4 compositions following lyophilization was very high and nearly equivalent to the fresh samples.

### vi. Uptake of Trehalose by Isolated Cells in Suspension vs. Embedded Placental Cells

Previous reports of lyophilization of cells in suspension have used trehalose as a lyoprotectant. To confirm that placental cells can uptake trehalose, cells were fully isolated from chorionic membranes and added to a well-plate. To the cell suspension, a commercially available fluorescently tagged trehalose, FITC-trehalose, was added and allowed to incubate for 4 hours at room temperature. Cells were washed multiple times to remove any remaining free FITC-trehalose. Figure 16 shows data confirming that placental cells in suspension are able to uptake trehalose at low levels.

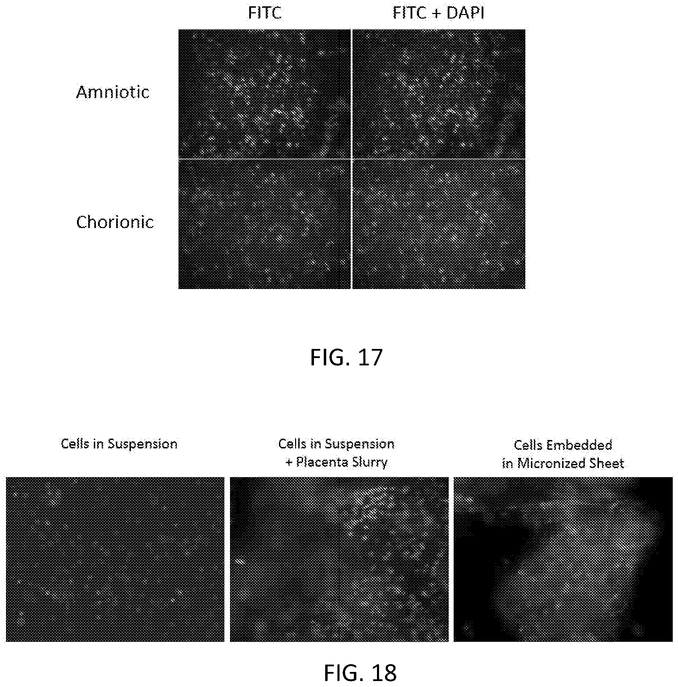

To further investigate the protective mechanism that leads to viable lyophilized placental membrane compositions, fresh amniotic and chorionic membranes were incubated in 2.5 mM FITC-trehalose in a 0.5M trehalose solution at room temperature for 1 hour and then thoroughly washed to remove excess trehalose. Next, membranes were stained with the cell nucleus stain, DAPI, to distinguish cells that took up FITC-trehalose from cells that did not. Cells embedded in amniotic membranes and chorionic membranes both readily uptake trehalose in the short incubation time (Figure 17). Longer incubation times with higher concentrations of trehalose at an elevated temperature could increase diffusion of trehalose into these cells and may promote enhanced cell survival following lyophilization.

### vii. Investigation into the Role of Tissue Matrix on Cell Survival During Lyophilization

Given the above data, further investigation into the role of matrix on cell survival during lyophilization was warranted. In prior studies, placental cell suspensions (fully isolated from tissue) were soaked and lyophilized in a trehalose solution, but viability after rehydration was low (< 30%; Figure 18, left). To see if cell suspensions lyophilized in the presence of tissue matrix might better survive lyophilization, fresh isolated chorion stromal cells were mixed with a devitalized placental tissue slurry and incubated in the presence of trehalose for 3 hours to allow cells to attach to the matrix proteins and allow trehalose to diffuse around and into cells. After lyophilization, viability of cells in suspension was still very low (< 10%; Figure 18, middle), which starkly contrasts the viability of cells embedded in tissue matrix of viable lyophilized chorionic membrane compositions (Figure 18, right). This result indicates that placental cells never removed or isolated from native tissue matrix appear to be better protected from lyophilization than placental cells isolated and in suspension.

### viii. Compositions of Viable Lyophilized Cartilage Allograft

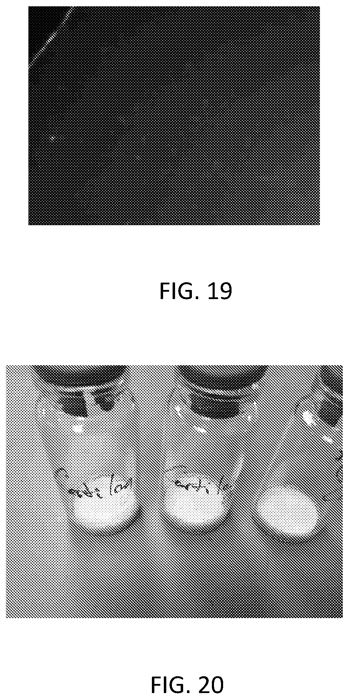

The same lyophilization methods effective for placental membrane and skin allograft compositions were used for cartilage graft compositions. First, intact pieces of bovine cartilage were incubated with FITC-trehalose for 4 hours and washed thoroughly to remove excess trehalose. As shown in Figure 19, some trehalose can be taken up by chondrocytes embedded in the dense collagen type II-rich matrix of articular cartilage. Intact bovine cartilage, intact bovine cartilage with 1 mm pores, and micronized bovine cartilage were soaked in a trehalose solution for 4 hours at 37C with agitation prior to lyophilization in the same trehalose solution.

After lyophilization, intact cartilage pieces no longer had an opaque white color, but appeared translucent. Upon rehydration, the opacity of the cartilage compositions returned within 5-10 minutes. The lyophilized micronized cartilage compositions formed a somewhat rigid structure shown in Figure 20.

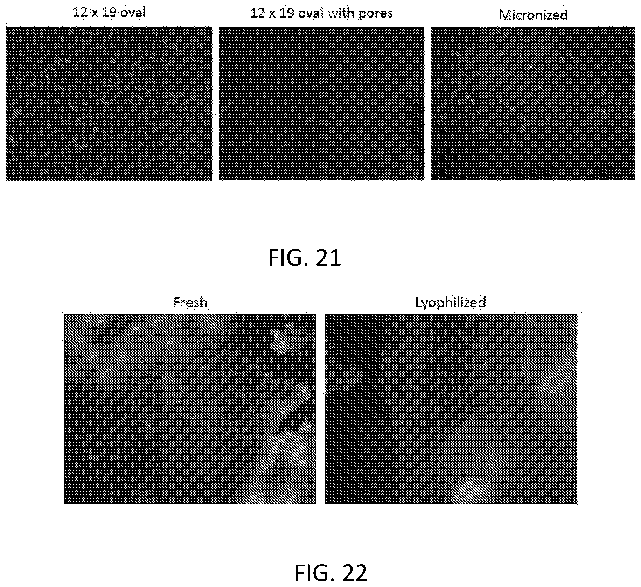

Only the micronized cartilage composition had viable cells (Figure 21), likely due to the shorter diffusion path for trehalose to individual chondrocytes embedded in the cartilage matrix. Diffusion of trehalose into the tissue matrix, and into the cell, likely plays a role in the mechanism of cell survival for lyophilized cartilage compositions, as well as other lyophilized compositions discussed herein.

### ix. Compositions of Viable Lyophilized Bone Grafts

The same lyophilization methods effective for placental membrane and skin allograft compositions were used for bone allograft compositions. Bovine bone particles of varying size ranges were produced and soaked in 0.5M trehalose solution for 4 hours at room temperature, then lyophilized in the same 0.5M trehalose solution. As shown in Figure 22, high cell viability could be retained in viable lyophilized bone graft compositions that was equivalent to the viability of fresh bone graft particles.

### B. Example 2

### 1. Viability data of lyophilized amniotic membrane after 90-days

### i. Experimental Plan:

1. The lyophilized 3 X 4 amnion was recovered from the sealed bag and rehydrated in DI sterilized water for 20 minutes
2. Upon complete rehydration, the AM was introduced into a 40 fold dilution of dispase solution for 2 minutes at RT. Using program spleen 2 on GentleMACS, the cells were recovered out of the rehydrated AM.
3.The whole solution from the gentleMACs tube was passed through 100um cell strainer to obtain cells.
4.The cells suspension was then spun down at 2000rpm for 5mins in a falcon tube to wash off dispase.
5.The supernatant was discarded and the resulting pellet was reconstituted in 80ul of DPBS.
6.40ul of cell suspension was added to 40ul of trypan blue to count the live and dead cells using hemocytometer. 2 operators counted the cells independently.
7.40ul of the remaining cell suspension was incubated in Calcein AM and EtBr solution for 10minutes.
8.The cell suspension in Calcein AM and EtBr solution was washed by spinning down the cells and resuspending the cells in 40ul BPBS.
9.The cell suspension was spread on a slide covered by a coverslip and then imaged using the Evos FL auto microscope.

### ii. Results:

Isolated cells stained with trypan blue were counted by two independent personnel and averaged. The percent live cells in the isolate (from the sample under study) averaged at 66% after 90 days.

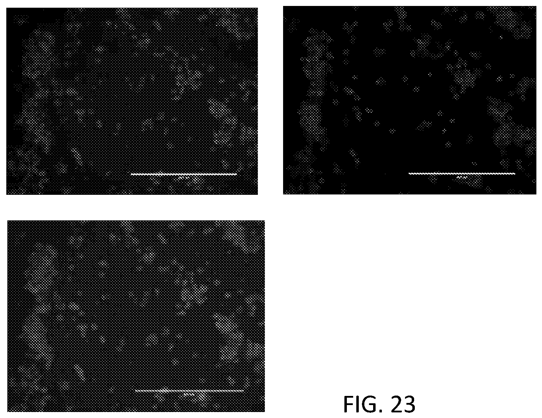

Using the live dead stain, isolated cells stained with Calcein AM and EtBr solution were imaged as shown in Figure 23.

This staining confirms the results for cell viability quantitation using trypan blue staining and shows that cells isolated from a viable lyophilized amniotic membrane that was stored at room temperature for 90-days remain viable and stable for at least three months. Images for the green channel (Calcien AM, viable) and red channel (EtBr, dead) are included, along with the overlaid image for both channels, for two different fields of view of the same sample.

### iii. Discussion:

Viable cells post 90 days (stability of lyophilized product) was observed in 0.25M trehalose treated amnion undergoing lyophilization and sealed immediately post retrieval of the sample from the lyophilizer.

### 2. Effect of antioxidants in combination with trehalose on cell viability after lyophilization

The purpose of this experiment was to assess two different formulations and their effects on lyophilization of AM using the same lyophilizing parameters.

### i. Experimental Plan:

### a. Trehalose and Catechin Solution

10.Prepare 0.25M Trehalose solution (T).
11.Prepare 1mg/ml Catechin in 0.25M Trehalose solution (T+C)
12.Incubate one 5 X 4 cm sq amnion in (T) for 40 minutes on a shaker at room temperature
13.Incubate one 5 X 4 cm sq amnion in (T+C) for 40 minutes on a shaker at room temperature
14.The amnions were removed from the T and T+C solution and placed between medical grade gauze and placed onto a 96 well place cover.
15.The plate covers with the amnion were placed in the lyophilizer, doors securely closed, and run on the program.
16.Upon completion of lyophilizer program, vacuum was released and dry AM was recovered.
17.The dry AM was rehydrated in DI sterilized water for 15 minutes.
18.A second dry AM was sent for residual moisture content analysis
19.The rehydrated AM was placed in a solution of ethidium bromide and Calcein AM, both at a concentration of 1:1000. DAPI stain at a concentration of 1:4000 was also included. The membranes were incubated at room temperature for 10 minutes, washed and then imaged using Evos FL Auto microscope system.
20.Images collected were superimposed using ImageJ.

### ii. Results

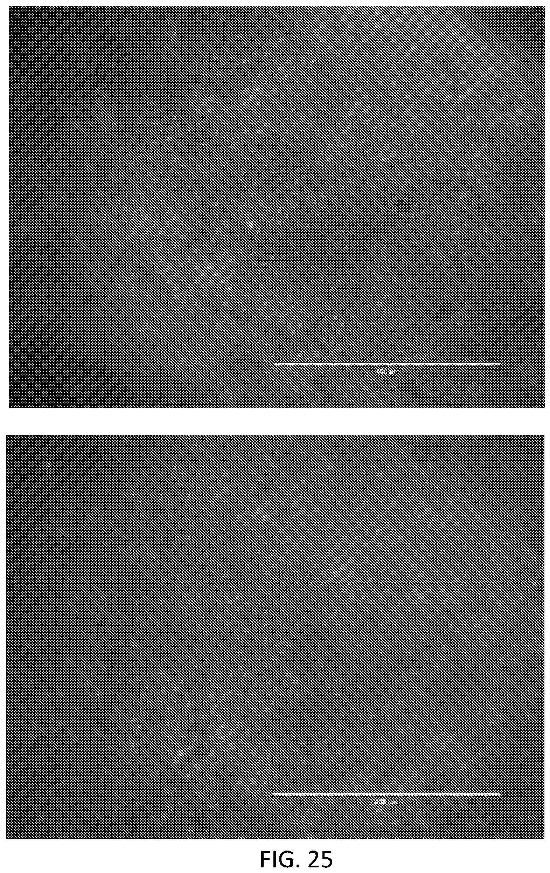

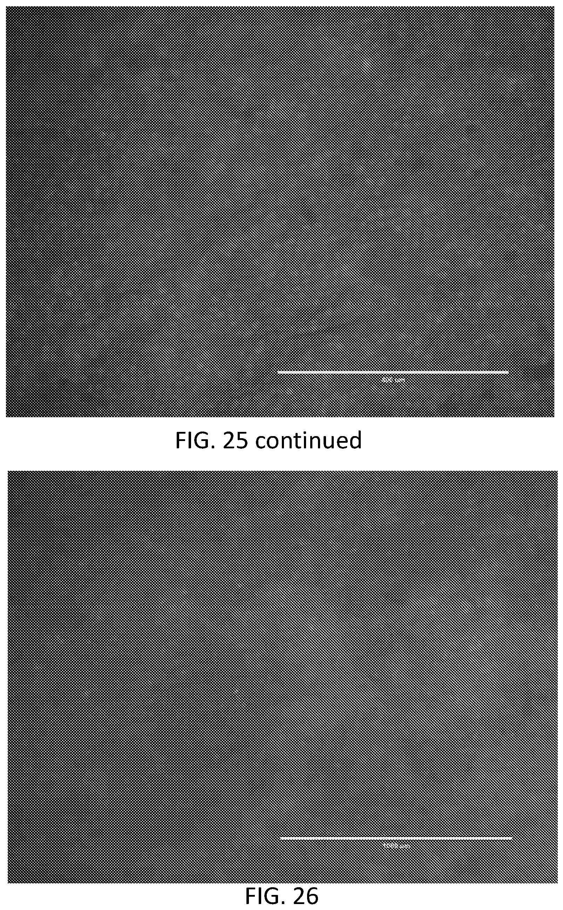

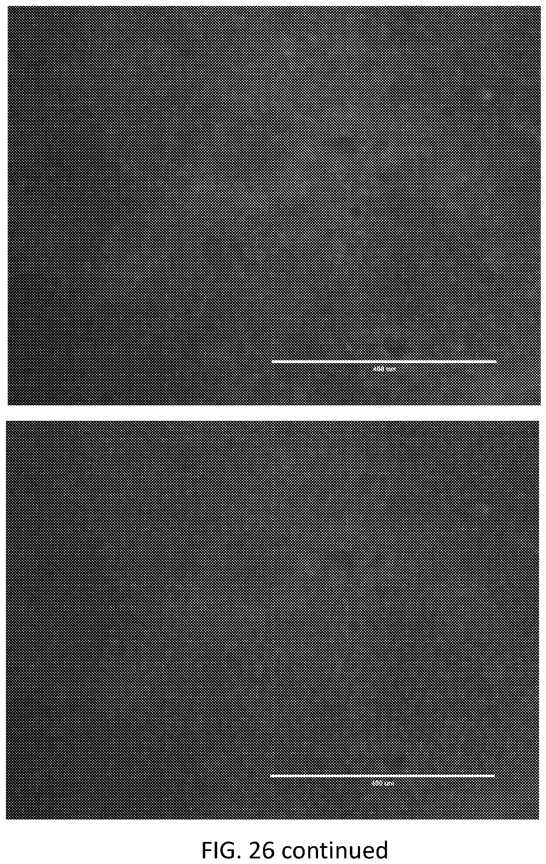

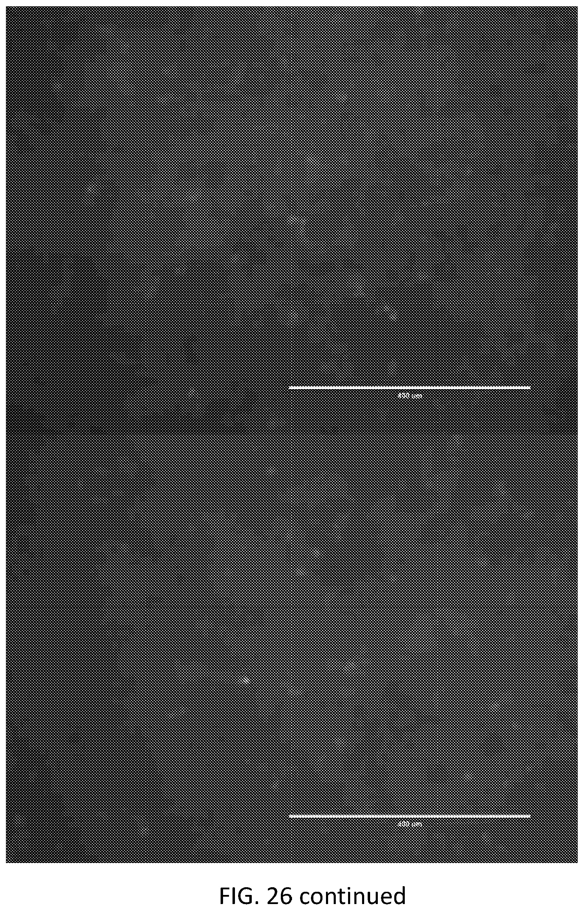

Dry AM after lyophilization is shown in Figure 25 and 26.

### iii. Discussion:

Catechins belong to a category of compounds known as flavanols and are found only in foods and drinks derived from plants. In these studies we observed that the incubation of amnion in Trehalose solution followed by lyophilization resulted in retention of viable epithelial cells. However, the inclusion of Catechin in the trehalose solution in which the amnion was incubated and then lyophilized resulted in the retention of both viable epithelial and viable stromal cells. The possible explanation of the inclusion of catechin in improving cell viability post lyophilization could be the action of antioxidant properties of catechin.

### 3. Viability of Isolated Amniotic Epithelial Cells after Lyophilization

The purpose of this experiment was to evaluate the effects of lyophilizing placental tissues using trehalose in conjunction with epigallocatechin gallate or catechin (USP grade) all in Tris buffer.

### i. Protocol:

### a. Tissue Sheet Preparation:

1.Process and separate amnion, chorion, and umbilical cord tissues with overnight antibiotic soak.
2.Deposit tissues in 50 ml conical tubes and add 10 ml of solution according to the following chart. Create three samples for each solution:

| | |
|---|---|
| **Trehalose (standard lyophilization solution control)** | 0.25 M trehalose in 20 mM Tris buffer |
| **Trehalose plus EGCG** | 0.25 M trehalose and 1.0 mg/ml EGCG in 20 mM Tris Buffer |
| **Trehalose plus Catechin** | 0.25 M trehalose and 1.0 mg/ml Catechin in 20 mM Tris Buffer |

3. Soak tissues at 4° C for >1 hr before lyophilization.
4.Cut tissues (both amnion and chorion) into 5x5 pieces.
5.Place tissues between 2 pieces of medical gauze and place within an aluminum pouch.
6.Lyopholize using prescribed program.

### ii. Minced Chorion Preparation:

1.Process and separate amnion, chorion, and umbilical cord tissues.
2.Deposit tissues in 50 ml conical tubes and add 10 ml of solution according to the following chart. Create three samples for each solution:

| | |
|---|---|
| **Trehalose (standard lyophilization solution control)** | 0.25 M trehalose in 20 mM Tris buffer |
| **Trehalose plus EGCG** | 0.25 M trehalose and 1.0 mg/ml EGCG in 20 mM Tris Buffer |
| **Trehalose plus Catechin** | 0.25 M trehalose and 1.0 mg/ml Catechin in 20 mM Tris Buffer |

3.Soak tissues at 4° C for >1 hr before lyophilization.
4.Mince soaked chorion using mezzaluna in a glass dish until minced tissue can be withdrawn via a 20 g syringe
5.Add 1-2 ml of minced chorion into glass vials for lyophilization with vented caps
6.Prepare 2 samples of minced chorion for an additional two vials with normal caps and store at 4° C for long-term stability testing. These samples will be placed within the cold room to check for viability later
7.Lyophilize using prescribed program.

Samples are later rehydrated in H₂O. Live/Dead staining is performed on samples and images are obtained and perform "autocounts" using Evos microscope.

### iii. Discussion:

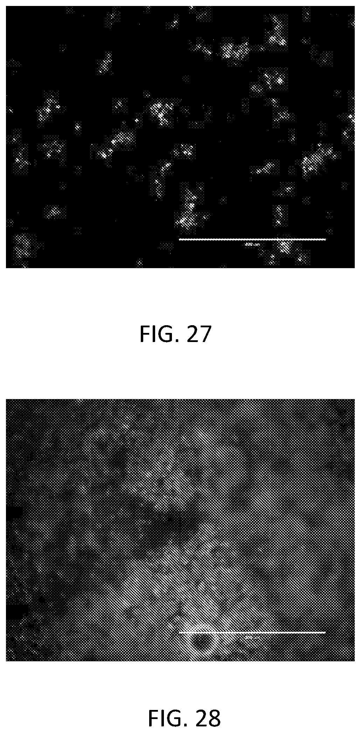

Dried tissue "sheets" and cells in all groups appeared to demonstrate approximately 50-60% viability after Live/Dead staining (Figures 27-30).

### iv. Results:

Figures 26 and 27 show the retention of cell viability after lyophilization of amniotic membranes using lyoprotectants solutions containing trehalose with or without the antioxidant catechin. Figure 26 shows that the epithelial cell viability of the amniotic membrane is relatively high (60-80%) when using a solution with trehalose alone (3 different fields of view are shown). Figure 27 shows images of amniotic membranes from the same donor (starting material) that were prepared using a lyoprotectants solution containing trehalose and catechin. The viability of the epithelial layer (first 3 images) and stromal layer (last 2 images) are higher than membranes shown in Figure 26 and estimated to be 80-95% viable in both layers.

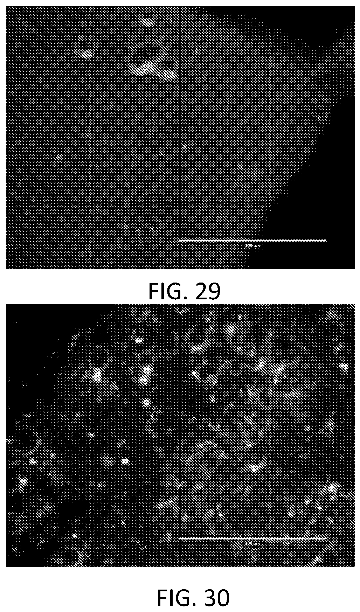

Figure 28 shows that small clusters of isolated amnion epithelial cells (2 or more cells still adjoined by matrix proteins or cell-cell junction proteins) also stay alive after lyophilization when prepared with a trehalose + catechin solution. Figure 29 shows the cell viability for micro-sheets of amniotic membrane that was prepared with a trehalose + catechin solution that have 50-60% viability. Figure 30 shows the cell viability for minced chorionic membranes treated with trehalose + catechin that have 70-80% viability after rehydration. Cell viability for minced chorionic membranes treated with trehalose + EGCG that have 80-90% viability after rehydration. Inclusion of an antioxidant with trehalose may improve cell viability after rehydration and/or promote long-term stability of the compositions.

### C. Example 3 (Process Development for Manufacturing of Viable Lyopreserved Amniotic Membrane (VLAM) Products)

This example provides a detailed description of the critical process parameters and development of the robust manufacturing process for a viable lyopreserved amniotic membrane (VLAM) using a 24 hr. lyophilization cycle on a large scale lyophilizer (Lyostar2). The process parameters initially defined and assessed in feasibility studies were re-evaluated in this study using a 24 hr. lyophilization cycle. This study demonstrates that the optimized manufacturing process results in a VLAM product that meets all pre-set cell viability, residual moisture, handling properties and sterility specifications. VLAM is stable after 3 months storage at room temperature: it continues to meet pre-set specifications without loss of cell viability or change in residual moisture content.

Cryopreservation is currently the only method for long-term storage of living cells and tissues. However, cryopreservation requires specialized ultra-low temperature storage equipment that limits widespread use of products containing living cells. To address this limitation, a lyopreservation technology has been developed that allows for ambient storage of living cells and tissues. This method can be applied to many different cell and tissue types, including placenta, skin, bone and cartilage.

Previously, it has been shown that amniotic membrane (AM) processed using this lyopreservation technique (VLAM) retains endogenous viable cells, as well as structural and functional properties of fresh and cryopreserved AM.

### 1. Methods

### i. VLAM Acceptance Criteria

Table 4 describes acceptance criteria for VLAM tests that were utilized during this study.

**Table 4. VLAM Test Acceptance Criteria**

| **VLAM Test** | **Method Description** | **Acceptance criteria** | **Comments** |
|---|---|---|---|
| Cell Viability | Quantitative | ≥70% | This is current lot release acceptance criterion for the amniotic membrane product and Core (cryopreserved placental membranes) |
| | Digestion of Amniotic and Chorionic Membranes for the Determination of Cell Counts and Viability Using the Trypan Blue Dye Exclusion Method | | |
| | Qualitative | "majority of the cells are viable" >50% viable cells | This criterion is used only for the process development |
| | Microscopic assessment of fluorescent tissues after staining usig the Live/Dead Cytotoxicity/Viability Assay Kit, Invitrogen | | |
| Epidermal Growth Factor (EGF) | Measurement of Epidermal Growth Factor (EGF) in Human Placental Membrane Lysates by ELISA | ≥7.8 pg/mL | This is current lot release acceptance criterion for the amniotic membrane product and the chorionic membrane product (cryopreserved placental membranes) |
| Residual Moisture | The Karl-Fischer colorimetric titration method | ≤ 9.70% | The test is performed by an external qualified vendor |
| Appearance post-lyophilization | Visual Inspection | No tissue rupture or cracks | This criterion is used only for the process development and by QC personnel for units selected for other tests |
| Adhesion to the mounting mesh | Physical separation of lyophilized tissue from the mounting mesh | Easy detachment from the mounting mesh as a single tissue unit without breaks or cracks | This criterion is used only for the process development and by QC personnel for units selected for other tests |

### ii. Tissue collection and Processing

Human full term placentas were provided by National Development and Research Institutes (NDRI), Anne Arundel Medical Center (AAMC), Gencure or Lifeline tissue banks from eligible donors after obtaining written informed consent. Placentas were processed according to procedures established at Osiris Therapeutics (Osiris Notebook 1006) and placed into antibiotic solution. Depending on the purpose for each experiment conducted in this study the processing of tissue post-antibiotic solution might differ from the established procedures**.**

### iii. Confirmation of Feasibility Results for Process Development using a Lyostar2 lyophilization cycle

The goal of the following experiments was to confirm suitability of method parameters described in RR16005 for transition to a new lyophilizer and an optimized lyophilization cycle. Table 5 summarizes parameters developed and described in RR16005. Cell viability was utilized for evaluation of each parameter.

**Table 5. Key parameters evaluated in RR160005.**

| **Key Parameters** | **Experimental Conditions** | **Conclusions*** |
|---|---|---|
| **Lyopreservation solution composition** | • cryopreservation solution (5% HSA, 10% DMSO, and 70% Saline) | A 0.5 M trehalose in DPBS lyopreservation solution was chosen. |
| | **•** 25% HSA in DPBS | |
| | **•** 0.1 M Trehalose in DPBS | |
| | **•** 0.25 M trehalose in DPBS | |
| | **•** 0.5 M Trehalose in DPBS | |
| | • 1.0 M Trehalose in DPBS | |
| **Tissue incubation time in the lyopreservation solution** | • 1 hour | A minimal of 1 hour tissue soaking in the lyopreservation solution was acceptable |
| | • 2 hours | |
| | • 3 hours | |
| | • 24 hours | |
| **Packaging Configuration** | • Borosilicate glass vial | The Tyvek pouches were chosen as the optimal option and used in further experiments. |
| | • Plastic Tray | |
| | • Plastic Tray with self-sealing pouch | |
| | • Tyvek pouch with Tyvek header outer pouch | |
| **Graft Mounting Material** | • Plastic backing | Extruded polypropylene mesh was selected based on tissue quality, handling properties and ease of use (in addition to cell viability) |
| | • Woven polypropylene mesh | |
| | • Extruded polypropylene mesh | |

| | | |
|---|---|---|
| *- based on qualitative VLAM cell viability using microscopic assessment of fluorescent tissues after staining with the Live/Dead Cytotoxicity/Viability Assay Kit, Invitrogen | | |

### iv. Lyopreservation solution composition

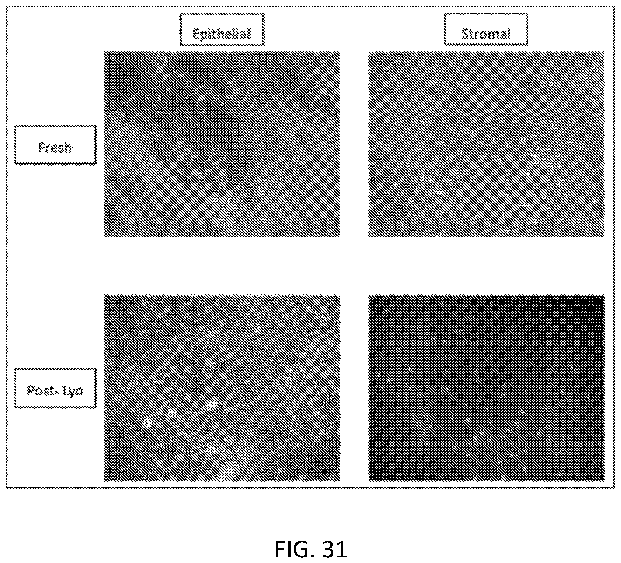

The goal of this experiment was to evaluate cell viability of VLAM using the 0.5 M trehalose in DPBS as a lyopreservation solution after lyophilization in FTS LyoStar II with an optimized lyophilization cycle. Following antibiotic incubation, AM was incubated with 0.5 M trehalose in DPBS for 60 minutes, packaged and lyophilized. The presence of viable cells in VLAMs was qualitatively assessed using the Nikon ECLIPSE TE300 microscope after VLAM reconstitution in the saline solution and staining with the LIVE/DEAD^{®} Viability/Cytotoxicity kit (Molecular Probes Inc., Eugene). Results showed that VLAM had greater than 50% viable cells (Figure 31). Quantitative assessment of cell viability was performed by QC personnel per QC312. Results demonstrated that VLAM samples met the cell viability acceptance criterion of >70% established for the amniotic membrane product, a cryopreserved placental membrane product. Data confirms that the 0.5 M trehalose lyopreservation solution is acceptable for the implementation in the VLAM manufacturing process.

### v. AM incubation time in the lyopreservation solution

Feasibility data (Table 5) supports 60 min AM incubation time in the lyopreservation solution prior to lyophilization. To confirm these results with the optimized lyophilization cycle, the 60 min incubation time was further tested in 0.5 M trehalose in DPBS. Testing of 105 min incubation was also included, which provides a time frame suitable for routine manufacturing. Three AMs derived from 3 different donors were used for experiments. Following antibiotic incubation, each AM was cut into two equal parts, one half was incubated in the lyopreservation solution for 60 min, and another half - for 105 min. After incubation in the lyopreservation solution, samples were packaged and lyophilized. As a control, cryopreserved AM samples were prepared. Prepared lyophilized and cryopreserved samples were submitted to QC for cell viability testing.

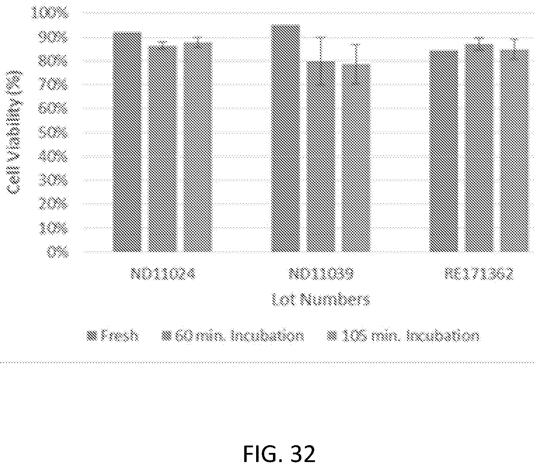

Results show that all samples met cell viability acceptance criterion of >70% (Figure 32). Mean cell viability for 3 lots of VLAM was 84.4% and 83.8% for 60 and 105 minutes incubation time, respectively. These results are in line with results previously reported. Data concludes that both 60 and 105 min incubation times are acceptable for an implementation in the VLAM manufacturing process.

### 2. VLAM Mounting Material and Packaging Configuration

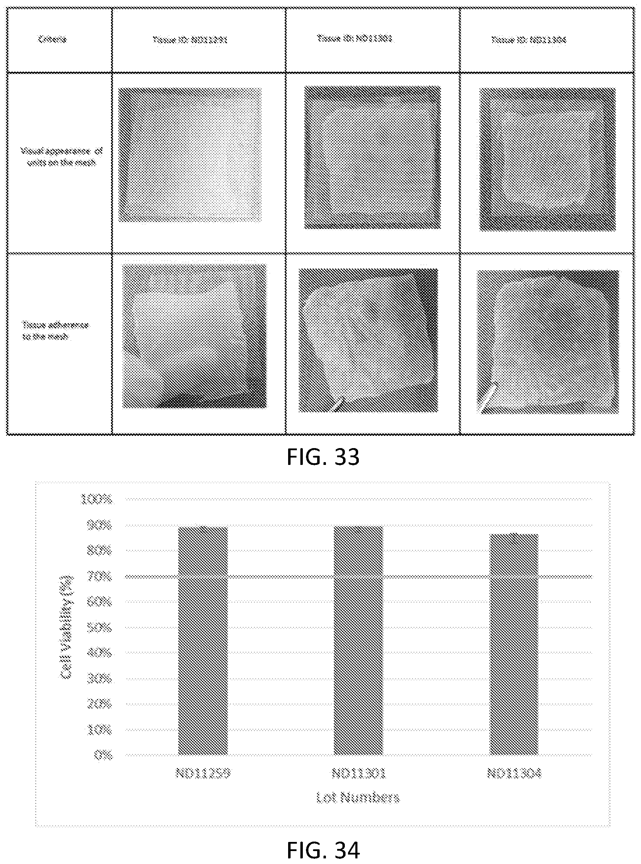

The key requirements for mounting material are: i) inert, non-toxic without leakage of components over time; ii) no change in handling properties during and post-lyophilization; and iii) no negative impact on graft characteristics and handling properties. The extruded polypropylene mesh (XN6080) per its specification and validation studies conducted by the manufacturers satisfies criteria listed above. The mesh was evaluated to confirm results of feasibility experiments: ease of mesh use, integrity of AM after mounting, adherence to the mesh and cell viability. AM grafts of 5x5 cm² from 3 donors were prepared, packaged, and lyophilized. Lyophilized samples were visually inspected for the presence of cracks and adherence to the mesh. Figure 33 shows visual appearance of VLAM mounted onto the extruded polypropylene mesh (XN6080). No tissue cracks and self- detachment from the mesh was observed. All lyophilized samples were submitted to QC and evaluated for cell viability.

A visual inspection of samples showed minimal tissue cracking (10% of samples) or self-detachment from the mesh, at the same time, when needed, the grafts can be easily detached from the mesh. Representative images of VLAM grafts are shown in Figure 33. Results confirm findings that XN6080 mesh is suitable for use in routine manufacturing of VLAM. Percent cell viability in VLAM mounted on XN6080 met the acceptance criterion of >70%. The mean % of cell viability for 3 samples derived from 3 different donors was 91.4% for one donor, 84.01% for one donor and 86.1% for one donor. Results are presented in Figure 34. In summary, all VLAM samples met visual inspection and percent viability acceptance criteria. Based on these results extruded polypropylene mesh was determined to be suitable for use in future experiments and routine manufacturing.

Packaging requirements for aseptic lyophilization are: i) should serve as a sterile barrier; ii) should allow moisture evaporation during a lyophilization cycle; iii) should serve as a moisture barrier after lyophilization. Tyvek primary (RLPR#531) and Foil with Tyvek header secondary pouch (RLPR#528) were identified as an acceptable packaging for routine manufacturing that meet the aforementioned requirements. These pouches maintain an exceptional moisture barrier with a low water vapor transmission rate (WVTR). The final configuration of the outer pouch consists of two materials: Symphony Foil (Roll Print Part# 26-1010) and ClearFoil X (Roll Print Part# 37-1304). Symphony Foil has a WVTR of 0.00 g/100 in² per day and ClearFoil X has a WVTR of only 0.004 g/100 in² per day. To confirm Tyvek pouches suitability, AMs were processed, packaged, and lyophilized. Lyophilized samples were evaluated for cell viability and residual moisture. To ensure that this packaging configuration acts as a moisture barrier post lyophilization, additional samples from this experiment were submitted for residual moisture analysis three months post lyophilization. For the packaging configuration to be acceptable all tested VLAM samples must pass acceptance criteria >70% cell viability. All samples met this acceptance criterion. The time zero and three months after lyophilization residual moisture content was ≤ 9.70% (Table 10). These results confirm results described in a feasibility study.

### i. Cell Viability Assay and VLAM Process Optimization

In preparation for cell viability testing, AM product units are digested with enzymes collagenase II and 0.5% trypsin with the purpose to release cells from tissue prior to staining with trypan blue followed by live/dead cell counting. However, for chorionic membrane (CM) products only collagenase II is used for sample preparation. For VLAM cell viability testing we used a combination of collagenase II and 0.5% trypsin since VLAM is an amniotic membrane. These experiments were designed to compare cell viability results when VCAM and VLAM sample are prepared without 0.5% trypsin vs the standard method (collagenase II and 0.5% trypsin). To test each condition, amniotic tissues after incubation in the antibiotic solution, then tissues were rinsed twice in DPBS. The amnion was cut into approximately equal two pieces. VCAM control grafts were prepared and stored at - 80°C until submitted to QC for cell viability testing. VLAM grafts were incubated in a 0.5 M trehalose solution for a minimum of 60 minutes, then mounted on extruded polypropylene mesh. All VLAM grafts were then transferred into Tyvek pouches prior to placing samples in a -80°C freezer for a minimum of 12 hrs. When ready VLAM samples were removed from the freezer and lyophilized using the 24 hr cycle. Both VCAM and VLAM samples from the same donor were submitted for the trypan blue dye exclusion cell viability assay with special instructions not to perform the trypsin digestions steps. Total and viable cell numbers were recorded and % of cell viability was calculated. For each digestion protocol mean % of cell viability values were compared. Standard deviations were calculated and a T-test was performed to determine whether there is a statistically significant difference for cell viability % between two methods of sample preparation.

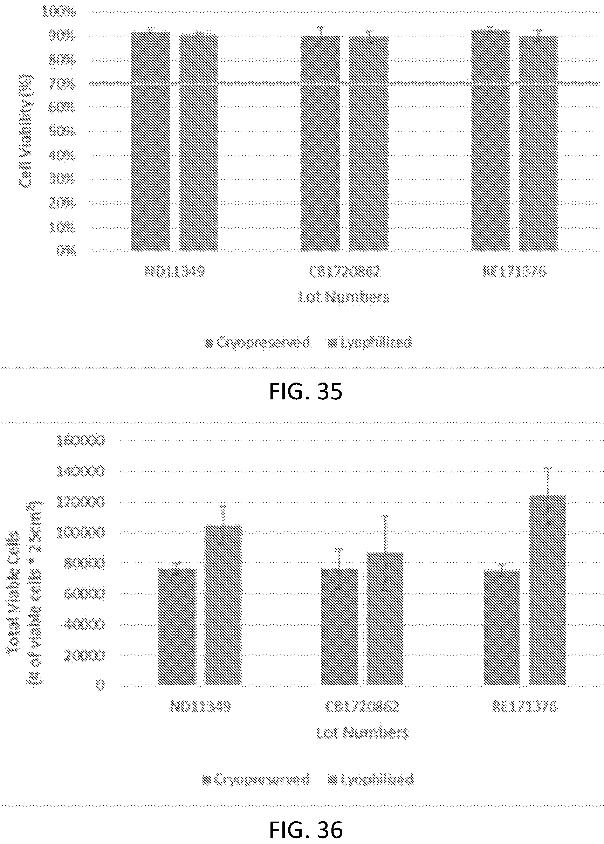

The average number of viable cells per mL in VCAM and VLAM samples and the cell viability % are presented in figure 35 and 36, respectively. The mean viable cell number per mL for VCAM and VLAM were 75922 ± 7045 and 105111 ± 23122 respectively. Percent viability was determined to be 91% and 90% for VCAM and VLAM, respectively. T-Test analysis demonstrates that there are no statistically significant differences in viable cell number (p= 0.1406) and % cell viability (p=0.5734) between two methods of sample preparation. Results conclude that collagenase II enzymatic digestion of AM without 0.5% trypsin is acceptable sample preparation method for the QC cell viability assay. Therefore, a collagenase II only for sample preparation is recommended to implement for routine quality control viability testing of the amniotic membrane product and VLAM products.

### ii. Lyophilization Parameters

### a. Duration of the primary drying phase of the 24 hr lyophilization cycle

The lyophilization cycle has three phases: freezing, primary drying and secondary drying. The first phase, freezing, transitions water in the product from liquid to solid. During primary drying water is sublimated from the product by increase in the temperature and decrease in vacuum pressure within the drying chamber. The point where approximately 95% of the water has been removed from a lyophilized substance/product is known as the endpoint of primary drying. Following primary drying, secondary drying is commenced to reduce further water remaining in the product through increasing temperature and vacuum pressures. The endpoint of primary drying can be determined through comparative pressure measurement (Pirani gauge vs. Capacitance Manometer). Throughout the drying step, the chamber capacitance monometer controls chamber pressure through measurement of the absolute pressure of the drying chamber. The Pirani gauge, which is also located in the drying chamber, measures pressure of the chamber through thermal conductivity of the gases within the chamber. During primary drying, i.e. when water vapor makes up a large percentage of the gas in the chamber, the reading of Pirani gauge will be approximately 60% higher than the capacitance manometer as water vapor thermal conductivity is approximately 1.6 times the thermal conductivity of air (at -20°C). As the vacuum increases in the drying chamber, the Pirani pressure increases due to the sublimation of water. The point on the lyophilization graph where Pirani pressure decreases sharply indicating the transfer of water from a gaseous state in the chamber to a solid state, ice, on the condenser is the start of bulk water removal from the product. It can be approximated that when the Pirani pressure is equal to the chamber capacitance monometer pressure (absolute chamber pressure) all gaseous water has been removed from the chamber and the primary drying stage is complete.

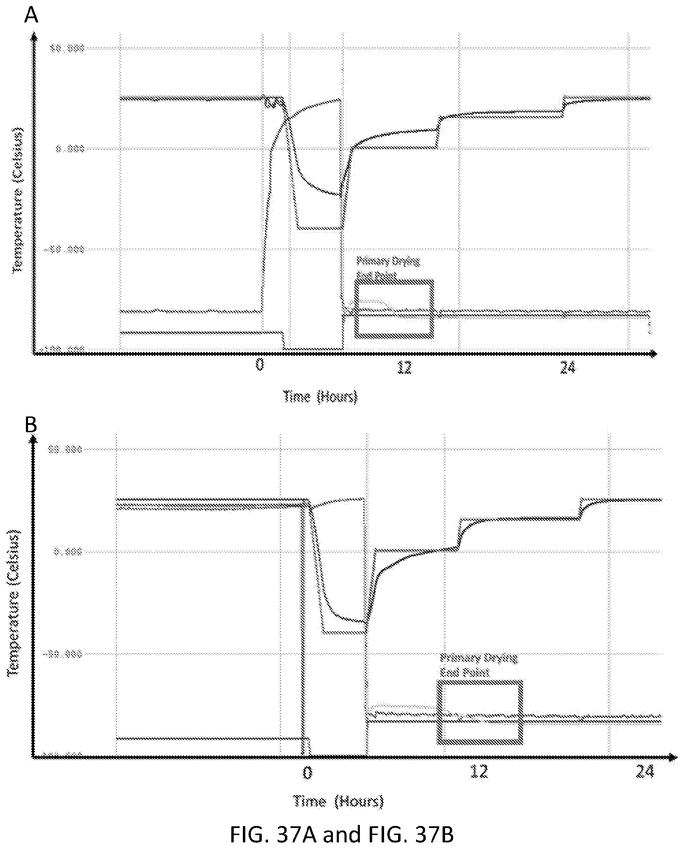

To define the primary drying endpoint for the 24hr lyophilization cycle with different unit number load graphical and numerical data for the Pirani pressure and the chamber capacitance monometer pressure were compiled and evaluated. Analysis demonstrates that the 24hr lyophilization cycle for 25 units achieves Pirani and capacitance monometer pressure equilibrium, i.e. the end point of primary drying, at approximately 12 hours into the cycle. Figure 37A shows this point (boxed) where Pirani gauge pressure (vertical lines at time 0 and approximately 7 hours) equalizes with the chamber capacitance monometer pressure. To confirm this result, an additional cycle with a total of 90 AM units was run. Figure 37B shows the cycle achieving the endpoint of primary drying for 90 units. The end of the primary drying was achieved after 12 hr for both 25 and 90 (maximal load) units (Figure 37 A and B).

### iii. Temperature Mapping in VLAM Unit Stacks During Lyophilization

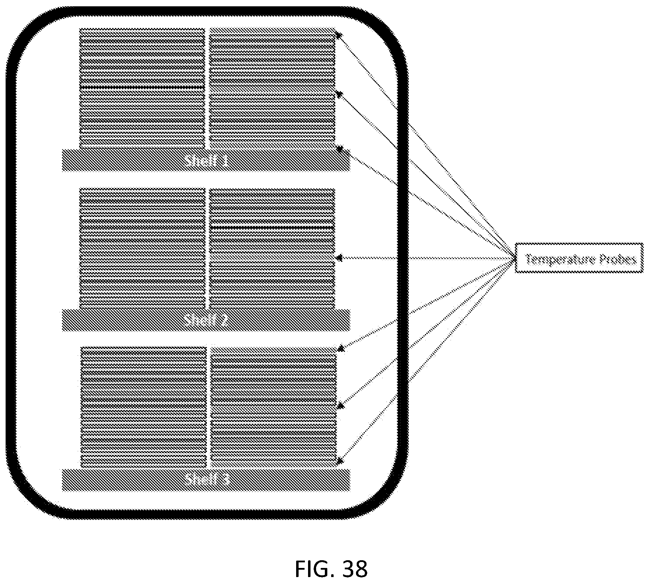

The parameters that define the rate of moisture removal are vacuum pressure, condenser temperature, and shelf temperature. These parameters and duration of the cycle play an important role in moisture removal. During lyophilization, thermal conduction from the shelf through a stack of a product may result in temperature differences throughout the stack of the product leading to variations in residual moisture content for product units within the stack. Therefore, uniformity of temperature for a specified quantity of AM units and loading configuration was determined. Lyophilization runs were conducted with different number of VLAM unit stack sizes (10, 15, 20, 30, and 40 units per stack) while tracking temperature throughout VLAM stacks using T-type thermocouples. AMs were processed, packaged and loaded into the lyophilizer shelves in various stack sizes. For some experiments, AM samples were stored in a -80°C freezer prior to lyophilization. T-type thermocouples temperature probes were positioned on the top, bottom, and middle of AM unit stacks (Figure 38). There are three shelves in the FTS Lyostar II. The bottom and the top shelves are closest and farthest from the condenser, respectively. The middle shelf being a center of the chamber contained a temperature probe positioned only in the middle of the stack (Figure 38).

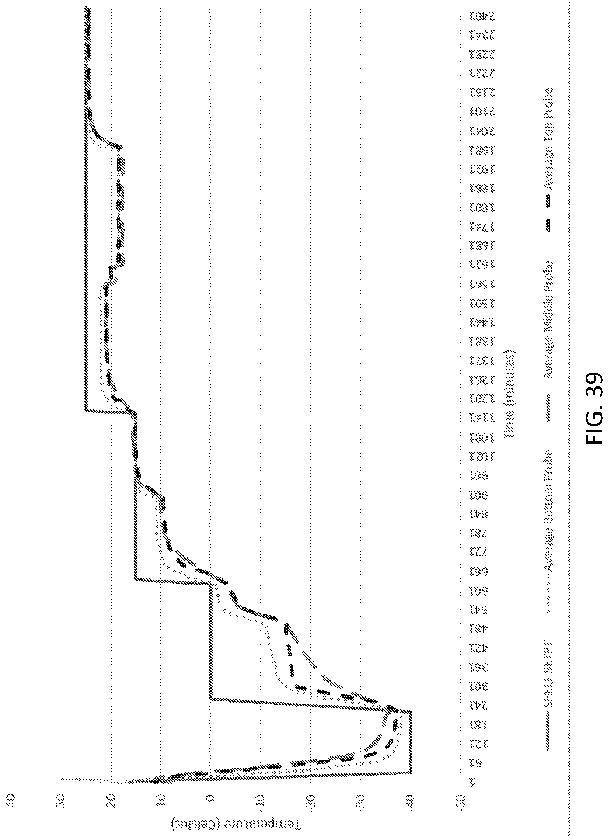

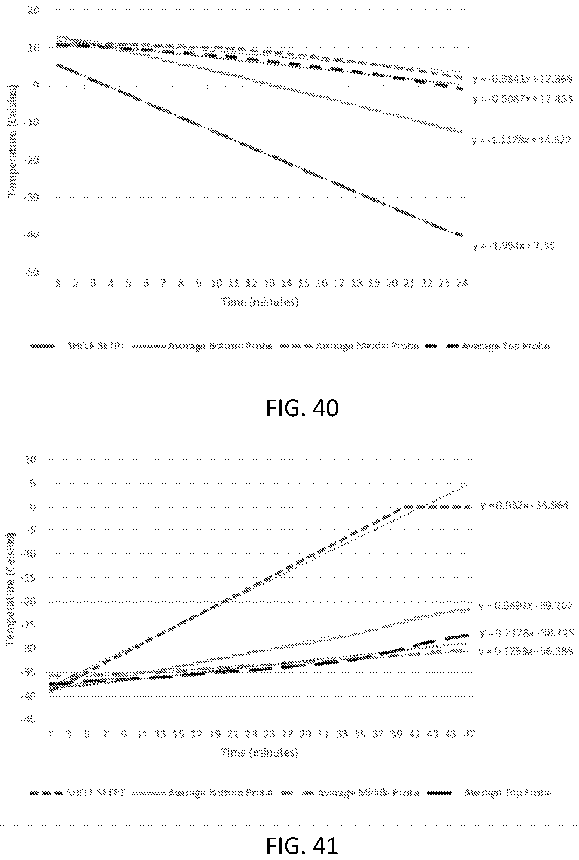

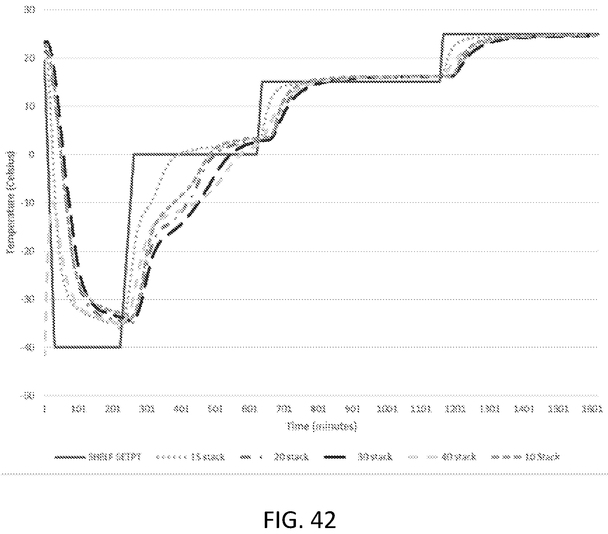

The lyophilization runs were commenced using the 24 hour part 2 - MRM cycle, and temperatures were recorded every minute throughout the cycle. The shelf temperature rate change (S(ΔT/min)) served as a control in this experiment (solid line on graph in figure 39). To determine the position in a stack (top, middle or bottom) that is the most thermally distinct from the control S(ΔT/min), an average temperature for each thermocouple temperature probe position at each time point combined for all stacks was plotted (Figure 39). Temperature is deviating more from the control at the middle position for VLAM stacks of all sizes in comparison to the control (shelf). This result defines the middle position as the most thermally distinct from the shelf control. This parameter is not dependent on VLAM stack size. To quantify the difference, the rates of temperature change per minute (ΔT/min) for each position in the stack was calculated for the freezing phase (Figure 40), and the heating during primary drying (Figure 41) phase of the lyophilization cycle. These phases were selected for evaluation because they represent the largest programmed temperature changes (Table 6). Averages of ΔT/min for freezing and heating steps during the primary drying phase were plotted (Figures 40-41). On each graph, a linear regression trend line and the corresponding equation are shown for each temperature probe and the shelf control. Using those linear regression curves, the slope was determined as Y=Mx + B, where M is equal to the ΔT/min for each temperature probe at the corresponding position in the stack. The control temperature rate changes (shelf) were 1.99°C/min during the freezing phase and 0.932°C/min during heating step of the primary drying phase. The temperature rate changes at the middle position of the middle shelf were -0.3841°C/min during the freezing phase and 0.1259°C/min during the heating step of the primary drying phase. This data confirms that the temperature rate changes at the middle stack position, on the middle shelf, is the most different from the control. This parameter is independent of the VLAM stack size. The temperature rate changes for the middle position were further investigated for VLAM stacks of different sizes. A number of units in the stack that shows minimal deviations in the temperature rate changes (ΔT/min) from the control at the middle position were evaluated for stacks contained 10, 15, 20, 30 or 40 VLAM units. Plotted graphs for the temperature rate changes for each stack were evaluated and compared to the control (shelf). Figure 42 shows temperature rate changes graphs for each stack size at different time points in the lyophilization cycle for the middle position of the thermal probes. A stack containing 15 VLAM units (red) has minimal deviation from the control temperature rate changes (blue) at all phases of the lyophilization cycle.

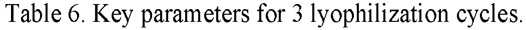

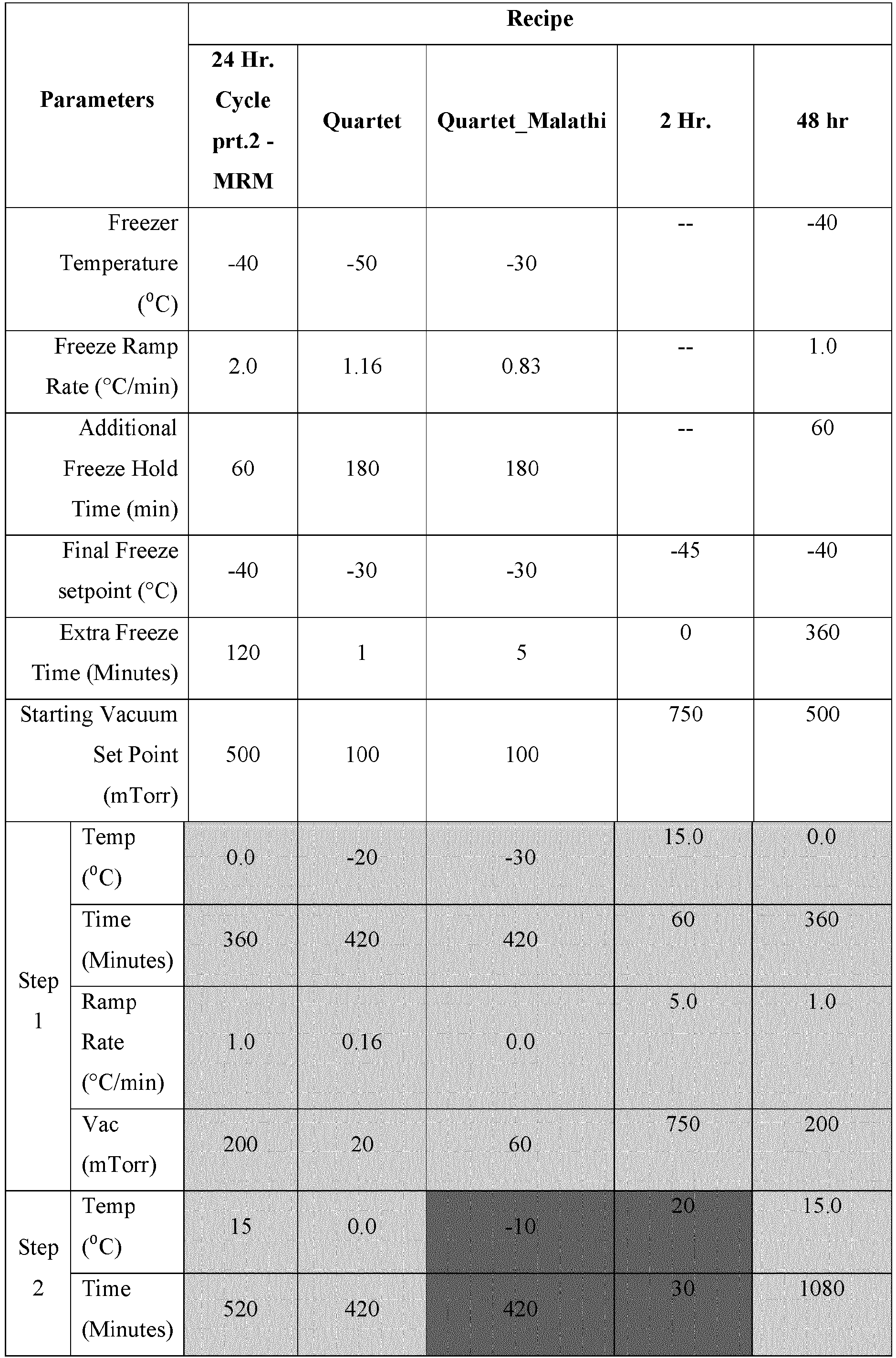

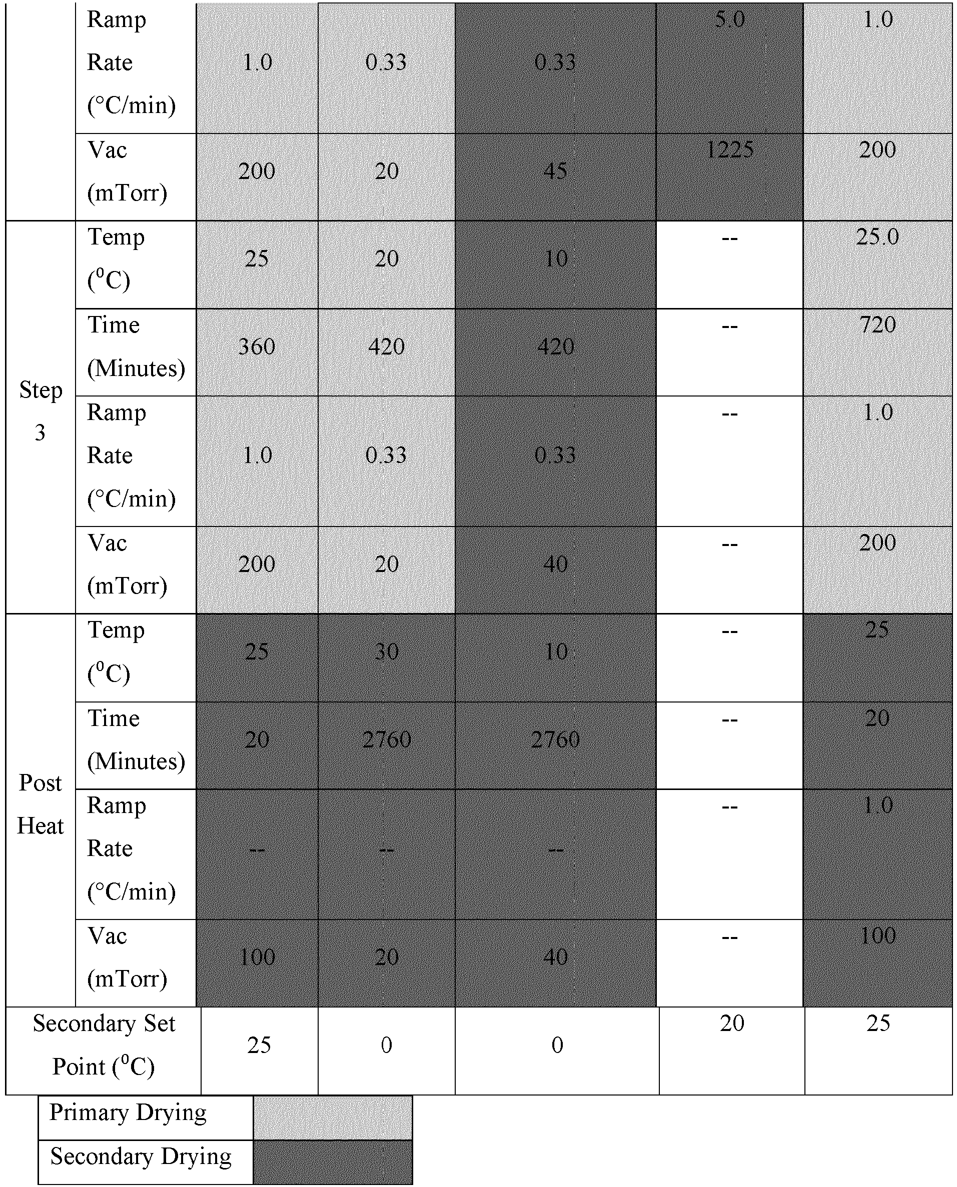

### iv. Suitability of the 24 hr lyophilization cycle for the VLAM manufacturing process

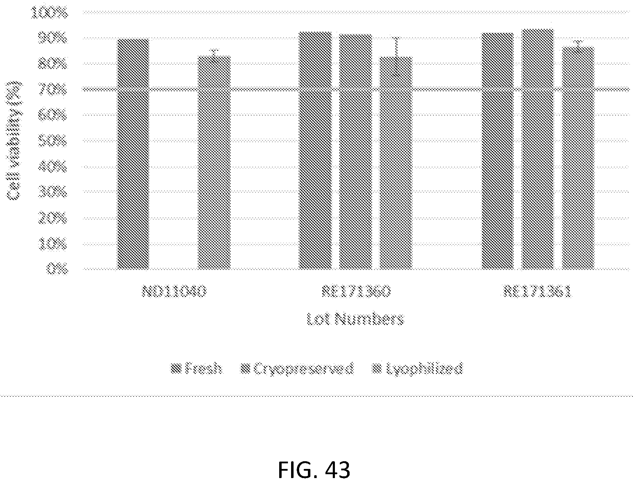

Parameters for the "Quartet" and "Quartet_Malathi" lyophilization cycles are described in table 6. These cycles were found to be suitable to preserve viable cells within the amniotic tissue. However, the lyophilizer Millrock L85 is not suitable for routine manufacturing due to its low capacity, poor parameter control and long duration of the cycle (72 hrs). The new OTI lyophilizer, FTS LyoStar II (SN#40274), is an appropriate model for routine manufacturing. It has high capacity, power and has better parameter control. A new cycle, "24 Hr. Cycle Part 2 -MRM" (Parameters are described in table 6), was developed and key parameters were defined. This cycle was evaluated using the FTS LyoStar II. Three AMs derived from 3 donors were included in the evaluation. Following antibiotic incubation, each AM was split into 3 parts. Each part was assigned to one of 3 groups: Group #1 - fresh AM, Group #2 - VCAM, Group #3 - VLAM. Fresh AM (Group #1) was treated with two DPBS rinses per BR07 and three samples per donor were submitted to QC per QC312 for cell viability testing. VCAM grafts (Group #2) were prepared per BR07 and stored in a - 80°C freezer. VLAM grafts (Group #3) were incubated in a 0.5 M trehalose solution for 60 minutes, then mounted on extruded polypropylene mesh and packaged into Tyvek pouches (RLPR528 & RLPR531). A total of 25 units were loaded into the lyophilizer FTS LyoStar II (SN#40274) for lyophilization with the 24 hr cycle ("24 Hr. Cycle Part 2 -MRM"). Three samples per group were submitted to QC for cell viability testing. Mean % of cell viability and standard deviation were calculated for each group. A t-test with p<0.05 was performed to determine whether differences in cell viability are statistical significance between groups. In addition, residual moisture content was tested after the 24 hr lyophilization cycle. The residual moisture was measured by Karl Fischer method according to AATB 2014 standards. The mean % of cell viability for fresh AM tissue was 91.23%. VLAM and VCAM samples had 84.01% and 92.15% of cell viability, respectively (Figure 43). All VLAM samples met the amniotic membrane product cell viability lot acceptance criterion of >70%. These results demonstrate that the 24 hr lyophilization cycle is deemed acceptable for the use in manufacturing based on cell viability test results.

In table 6, the end point of primary drying is the point were approximately 90%-95% of the moisture has been removed from the product as determined through the following processes: Techniques based on gas composition in the product chamber - comparative pressure measurement (i.e. Pirani vs capacitance manometer), dew point monitor (electronic moisture sensor), process H20 concentration from tunable diode laser absorption spectroscopy (TDLAS), Lyotrack (gas plasma spectroscopy); and others - product thermocouple response, condenser pressure, pressure rise test (manometric temperature measurement (MTM) or variations of this method).

The endpoint of primary drying or initiation of secondary drying can be the point where typically temperature and pressures are increased simultaneously. In some instances, only temperature can be increased to initiate secondary drying but is coupled with prolonged increased temperature exposure (i.e. Quartet-Malathi "post heat" step).

The lyophilization cycles and specific tissues of table 4 are as follows: Quartet - compatible with AM and umbilical tissue (UT); Quartet-Malathi - compatible with AM, UT; 48 hr cycle - compatible with UT, CM; 24 hr cycle prt 2-MRM - compatible with AM, UT; 2 hr cycle - compatible with AM, UT.

Table 7 provides lyoprotect solution compositions. Lyoprotectant soak times require a minimum of 1 hour soak time. A 24 hour soak time provided similar results to 1 hour soak time. At time points less than 1 hour, poor results were obtained regarding cellular viability retention.

**Table 7.**

| **Lyoprotectant Solution Composition** | **Cell Viability** | **Comments** |
|---|---|---|
| 10% DMSO, 12.5% HSA in D-PBS | <50% viable cells | Not selected |
| 25% HSA in D-PBS | No viable cells | Not selected |
| 0.25M Trehalose, 12.5% HSA in D-PBS | Majority of cells are viable | Not selected |
| 0.1M Trehalose in D-PBS | No viable cells | Not selected |
| 0.25M Trehalose in D-PBS | Majority of cells are viable | Inconsistent results |
| 0.5M Trehalose in D-PBS | Majority of cells are viable | Selected: highest cell viability by qualitative assessment with simplest composition |
| 1M Trehalose in D-PBS | Majority of cells are viable | Similar results to 0.5 M trehalose. |
| 0.25M Trehalose, 1mg/mL Catechin in D-PBS | Majority of cells are viable | Not selected, causes brown coloration of amnion |

### v. Pre-Lyophilization AM Treatment (incubation or rinse) in 0.045 M Trehalose Solution

Visual inspection of VLAM units in this study showed cracking and/or stickiness of the AM tissue to the XN6080 mesh for approximately 10% units per lot (1 out of 10 units). Cracking and stickiness of AMs is due to sugar (trehalose) accumulation on the surface of the tissue during lyophilization. As a simple method to reduce the amount of sugar from the tissue surface we tested a pre-lyophilization rinse of the AMs with 0.045 M Trehalose in DPBS after incubation of the tissue in 0.5M trehalose lyoprotectant solution. All samples after lyophilization were undergoing visual inspection and should pass the acceptance criteria described in table 4. In addition, VLAM samples from this experiment were tested for cell viability, and VLAM should have >70% viable cells for the rinse to deem acceptable. In this experiment, AMs from 4 different donors were used. Each AM after incubation in the 0.5M trehalose lyopreservation solution was split into two equal parts. One part (Group #1) was incubated in 0.045 M trehalose in DPBS for 2 hours, and Group #2 was rinsed four times in 0.045 M trehalose in DPBS. The 0.045 M Trehalose in DPBS solution was prepared by diluting 50 mL of 0.5 M trehalose in 500 mL of DPBS. Both groups were mounted, packaged, and lyophilized. After lyophilization, a visual inspection was performed on 100% of samples from both groups for each tissue. Three samples from each group were submitted for cell viability testing. A mean percent of cell viability and standard deviation were calculated for each group. A t-test was performed to determine whether there are significant differences in the percent of cell viability between the two experimental groups.

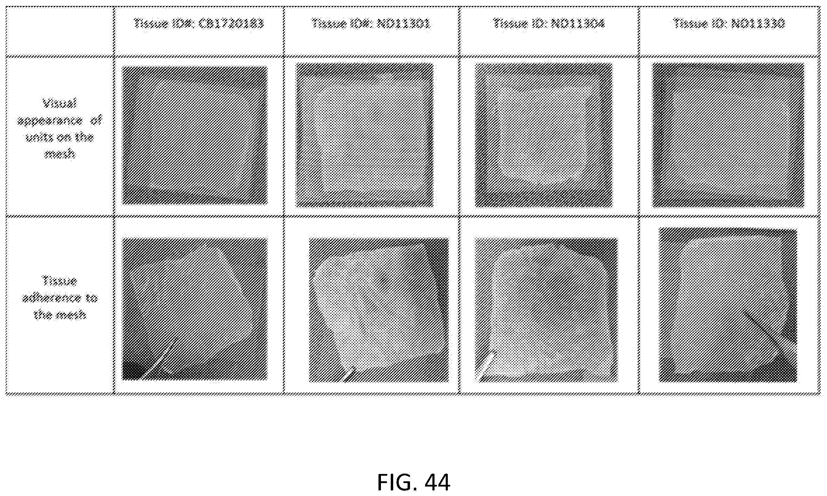

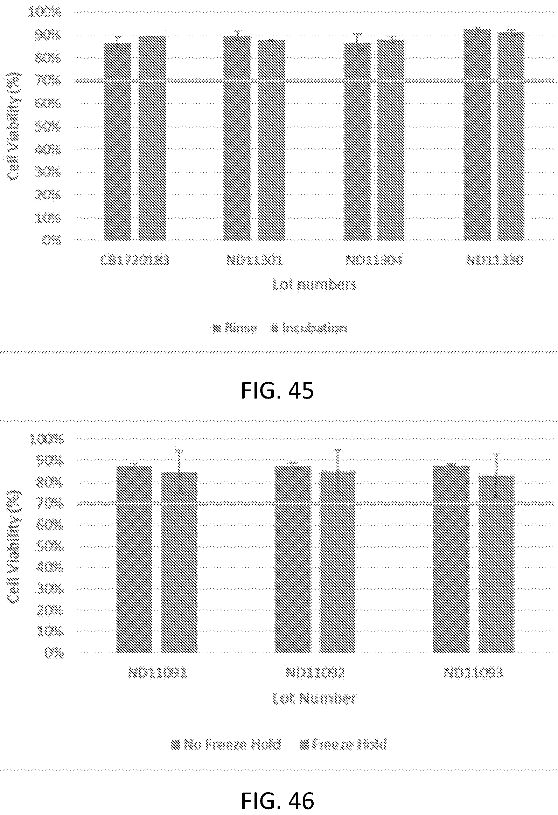

A visual inspection did not identify any VLAM units with tissue cracks or stickiness to the mesh. Representative images of VLAM samples are shown in figure 44. Results demonstrate that 2 hr incubation or 4 rinses in 0.045 M trehalose in DPBS pre-lyophilization prevent tissue cracking and/or stickiness to the mesh post-lyophilization. Results of cell viability testing are shown in figure 45. All samples met the acceptance criterion of >70%. The mean percent of cell viability for samples incubated in 0.045M trehalose was 89.76% ± 4%. The mean percent of cell viability for samples rinsed 4 times in 0.045 M trehalose was 89.08% ± 2%. There were no statistical significant differences between these two groups (p = 0.8327). These results indicate that the 0.045 M trehalose in DPBS solution is suitable for the use in future experiments and for the implementation in the routine manufacturing process with the purpose of reduction of tissue cracking and stickiness to the mesh after the lyophilization.

### vi. Intermediate "in process" pre-lyophilization storage -80°C

Storage of packaged unit at -80 C prior to lyophilization is a beneficial option allowing schedule flexibility for routine manufacturing. Therefore, in this experiment an effect of -80°C storage of packaged AM units on cell viability after lyophilization was investigated. Following antibiotic incubation, Each AM derived from three donors was split into 2 equal parts: one part (Group #1) was lyophilized immediately after packaging, and the units produced from the second part (Group #2) were placed into a 18x24 Poly bag (CS00160) after the packaging and stored at -80°C for 97 hours prior to lyophilization. After 97 hours at -80°C, AM grafts were removed from the 18x24 Poly bags (CS00160) and lyophilized using the 24 hr. cycle. Three lyophilized samples per donor were submitted to QC for cell viability testing. A mean percent of cell viability and standard deviation were calculated for each group. A T-test was performed to determine whether there are significant differences in the percent of cell viability between two experimental groups. The mean percent of cell viability for samples lyophilized immediately after packaging was 87.74% ± 1.11%. For sample stored at -80 C for 97 hr cell viability was 84.29% ± 3.01%. There were no statistical significant differences between these two groups (p = 0.1360). Results indicate that an intermediate "in process" AM unit storage -80°C for up to 97 hours has no negative impact on cell viability. Therefore, an intermediate storage at -80°C prior to lyophilization for a maximum of 97 hours is acceptable for use in routine manufacturing.

### 3. VLAM Manufacturing Process

A flow chart of the process and a step-by-step description are presented below.

### i. Processing

Process the placenta according to procedures used for the amniotic membrane product. Separate AM from other placental tissue and wash twice in DPBS . Incubate AM in ACD-A solution (11% ACD-A in saline) to loosen red blood cells. If needed, remove manually (using fingers) blood clots from the surface of the tissue. Wash AM twice in PBS. Then, incubate AM for 24 hours in antibiotic cocktail containing 50µg/mL Gentamicin sulfate, 50µg/mL Vancomycin, and 2.5µg/mL Amphotericin B in DMEM.

### ii. Packaging

Remove AM from antibiotic cocktail and wash twice in PBS. Incubate AM in 0.5 M Trehalose in DPBS solution for 60 to 105 minutes. Remove from 0.5 M Trehalose in DPBS solution and rinse in 0.045 M Trehalose in DPBS solution. Multiple AM rinses can be performed throughout mounting of grafts.

Using a template, cut the appropriately sized AM grafts from the whole membrane. Transfer the graft directly from the template to the mesh (XN6080 ). Place another piece of mesh (XN6080) to the top of the AM graft mounted to another piece of mesh (XN6080). Place graft into primary Tyvek pouch (RollPrint # RLPR531) and seal using AccuSeal heat sealer 540, 5300, or 5400 (or equivalent). The pouch can be sealed with the clear side of the pouch facing the heating element.

Place primary pouch in foil with Tyvek header pouch (RollPrint # RLPR528) and seal using AccuSeal heat sealer 540, 5300, or 5400 (or equivalent) along the Tyvek nearest the edge of the pouch. Ensure that the primary pouch is inserted far enough within the secondary pouch as to clear the Tyvek header providing sufficient space for final heat sealing. The pouch can be sealed with the symphony foil (metallic) side of the pouch facing the heating element.

Intermediate storage of AM units at -80°C. Packaged AM units can be lyophilized immediately or stored at -80°C for up to 97 hr prior to lyophilization. If lyophilizing the tissue without a -80°C storage continue to the transfer of packaged AM grafts in stacks of 15 units to the lyophilizer wherein the AM grafts can remain at room temperature prior to lyophilization for a maximum of 5 hours.

Transfer packaged AM grafts in stacks of 15 units into a 18x24 Poly bag and place directly into a -80°C freezer. AM units can remain in the freezer for a maximum of 97 hours.

Remove AM units from the -80°C freezer, and then from 18x24 poly bag. Load units into the lyophilizer. Maximal load is 2 stacks of 15 units per one lyophilizer shelf.

Lyophilization without intermediate unit storage at -80 C. Transfer packaged AM grafts in stacks of 15 units to the lyophilizer. AM grafts can remain at room temperature prior to lyophilization for a maximum of 5 hours.

Load units into the lyophilizer. Maximal load is 2 stacks of 15 units per one lyophilizer shelf and initiate the 24-hr lyophilization cycle protocol.

Remove units from the lyophilizer at the end of the cycle. Seal the secondary foil/Tyvek header pouch above the Tyvek header. Use cutter to remove Tyvek. Perform final packaging of the units. Store units at ambient temperatures.

**In** some aspects, the graft mount material can be mesh, basins, or plastic backings. Mesh has acceptable handling properties and acceptable cell viability. Basins have unacceptable handling properties and acceptable cell viability. Plastic backings have unacceptable handling properties and acceptable cell viability.

**In** some aspects, the packaging configuration can be glass vials, trays, self-sealing pouches, or tyvek dual pouches. Glass vials have acceptable cellular viability retention and unacceptable sterility retention. Trays have acceptable cellular viability retention and unacceptable sterility retention. Self-sealing pouches have acceptable cellular viability retention and unacceptable sterility retention. Tyvek dual pouches have acceptable cellular viability retention, acceptable sterility retention.

### 4. VLAM Characterization

VLAM units were prepared and characterized for the EGF content, cell viability and residual moisture.

### i. Evaluation of epithelial growth factor (EGF) presence in VLAM

VLAM samples derived from three donors were prepared. Lysates were prepared using the Qiagen TissueLyser LT. Results were evaluated using the current amniotic membrane product lot acceptance criterion: > 7.8 pg/mL EGF. Results are summarized in table 8.

**Table 8. EGF ELISA results for 3 samples representing 3 VLAM lots.**

| **Donor** | **Lys ate (pg/ml)** | **EGF (pg/ml)** | **EGF Mean (pg/ml)** | **Mean EGF per 3 Samples (pg/ml)** |
|---|---|---|---|---|
| **ND11329** | Sample 1 | 85.804 | 82.324 | 90.95167 |
| | | 76.745 | | |
| | Sample 2 | 74.499 | 74.938 | |
| | | 75.377 | | |
| | Sample 3 | 110.347 | 115.593 | |
| | | 120.612 | | |
| **ND11301** | Sample 1 | 118.245 | 119.467 | 86.98867 |
| | | 120.688 | | |
| | Sample 2 | 97.403 | 98.223 | |
| | | 99.042 | | |
| | Sample 3 | 42.601 | 43.276 | |
| | | 43.952 | | |
| **CB1720183** | Sample 1 | 176.803 | 179.83 | 202.136 |
| | | 182.858 | | |
| | Sample 2 | 218.468 | 225.575 | |
| | | 232.683 | | |
| | Sample 3 | 206.717 | 201.003 | |
| | | 195.29 | | |

### ii. Residual moisture content

There are three USP methods of determining residual water content, USP <921 Method I (Titrimetric), Method II (Azeotropic) or Method III (Gravimetric). Titrimetric, also known as Karl Fischer, requires the smallest sample by weight and cost effective. Residual moisture in VLAM 10 samples were prepared and shipped to WuXi App Tech for analysis. WuXi App Tech performed residual moisture analysis using the Karl Fischer, USP <921> .

**Table 9. Karl Fischer suitability results for the amniotic membrane product units.**

| **Karl Fischer Suitability Data** | |
|---|---|
| Tissue Lot Number | Residual Moisture (%) |
| ND10836 | 3.63 |
| | 3.9 |
| | 3.73 |
| | 1.53 |
| | 3.8 |
| | 3.64 |
| | 3.51 |
| | 4.03 |
| | 3.36 |
| | 3.69 |
| Average= | 3.70 |
| Standard Deviation= | 0.20 |

### a. Lot Acceptance Criteria

Since feasibility data has demonstrated the retention of viability at 12% residual moisture and that AATB regulations (2014 ed.) dictates that a residual moisture content range must be defined that does not impede tissue quality. An acceptable residual moisture content limit was defined through analysis of the results from multiple samples (n = 12). Residual analysis results from lyophilized AM using the 24 hour prt. 2 - MRM cycle are reported in table 10. The acceptable limit of residual moisture content was determined through calculation of the average content across the lots tested, (5.12%). The standard deviation was extrapolated from those results (1.53%). From these results an acceptable limit of residual moisture content was defined to be ≤9.7% residual moisture content. This limit is three standard deviations from the average calculated and will provide a buffer while ensuring the retention of tissue quality.

**Table 10. Residual Moisture content results provided by Karl Fischer Analysis and statistical analysis.**

| Tissue Lot Number | Residual Moisture Content (%) |
|---|---|
| ND11155 | 6.59% |
| | 6.24% |
| | 6.14% |
| ND11156 | 6.31% |
| | 6.19% |
| ND11039 | 7.25% |
| ND11291 | 4.18% |
| | 4.01% |
| RE171373 | 4.34% |
| | 2.95% |
| RE171375 | 2.87% |
| | 3.38% |
| ND11207 | 6.07% |

| | |
|---|---|
| Average | 5.12% |
| Standard Deviation | 2% |
| 3 Standard deviations | 4.58% |
| Upper Limit | 9.70% |

### b. Stability

Additionally, it is paramount that the residual moisture content remains constant from the time of initial testing until delivery so that therapies at time of application are appropriately representative of the product. An analysis of samples was performed to evaluate the impact residual moisture content provides regarding viability and stability. Lyophilized AM grafts were submitted for residual moisture content testing following lyophilization and final heat sealing was performed. An additional sample was submitted for analysis following a 2 month hold post lyophilization. Results are reported in table 11 showing initial residual moisture content of 7.25% whereas 2 months post lyophilization the residual moisture content was determined to be 6.07%. Both time points tested are acceptable, >9.70%. Additionally, three samples were submitted after a 3 month hold to determine the increase of residual moisture content over time and were compared against the known average of residual moisture content when tested immediately after lyophilization. Table 11 presents the data obtained and demonstrates that all samples tested pass residual moisture content acceptance criteria.

**Table 11. Residual moisture of VLAM. VLAM is evaluated for residual moisture content at time 2 months and 3 months post lyophilization. Two samples are evaluated for time point 2 months, initial (immediately post lyophilization) and 2 month. Three donors are tested for residual moisture content at time point 3 months.**

| Tissue Lot Number | Time Point Tested | Residual Moisture Content | Acceptable Limit | Pass / Fail |
|---|---|---|---|---|
| ND11039 | Initial | 7.25% | > 9.70% | Pass |
| | 2 month | 6.07% | | Pass |
| 888153177 | 3 mo. | 4.82% | | Pass |
| CB1606001903 | 3 mo. | 6.16% | | Pass |
| CB1606001904 | 3 mo. | 6.27% | | Pass |

### c. Cell Viability

VLAM samples derived from three donors were prepared. Samples were submitted for the trypan blue dye exclusion cell viability assay with special instructions not to perform the trypsin digestions steps. Results were evaluated using the current amniotic membrane product lot acceptance criterion: > 70% viable cells. Results are summarized in table 12.

**Table 12. Cell viability of VLAM. Mean % of cell viability ± SD from three donors (3 samples tested from each donor)**

| Donor | Mean Cell Viability (%) | Standard deviation (%) |
|---|---|---|
| N D11259 | 89.99% | 0.0195 |
| ND11301 | 89.50% | 0.0216 |
| N D11304 | 86.63% | 0.0368 |

### 5. Packaged AM pre-lyophilization stability

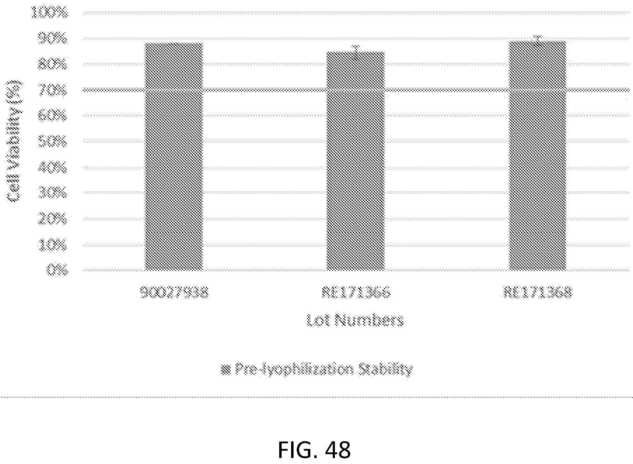

The purpose of this experiment was to define an acceptable "lag time" that can happen during the manufacturing process when packaged AM units can be transferred to a lyophilizer. Following antibiotic treatment, AM was split into two equal parts: One part (Group #1) - CVAM (control), and the second part of AM (Group #2) - VLAM packaged units that were kept on a bench for 5 hr prior to lyophilization. VCAM control samples were prepared and stored at -80°C until submission to QC for cell viability testing. VLAM samples were kept after packaging at ambient conditions for 5 hours and 20 minutes, and then units were placed in a lyophilizer. Three Group 1 VCAM and Group 2 VLAM units per donor were submitted to QC for cell viability testing. A mean percent of cell viability and standard deviation were calculated for each group. A T-test was performed to determine whether there are significant differences in the percent of cell viability between two experimental groups. All tested samples met the acceptance criterion of >70% of cell viability. The average percent of cell viability for Group 2 VLAM samples was 87.09% ± 3.77%. Group 1 control VCAM samples had in average 88.03% ± 0.93% cell viability. Results are shown in Figure 48. There were no statistically significant differences in cell viability between VCAM and VLAM samples (p = 0.6965). Results indicate that a delay of 5 hours and 20 minutes for packaged AM units prior to placuing them in a lyophilizer had no negative impact on cell viability. Therefore, during the manufacturing the acceptable "lag time" post packaging and transferring packaged AM units into the lyophilizer steps is up to 5 hours and 20 minutes.

### i. Defining a shipment temperature range for VLAM

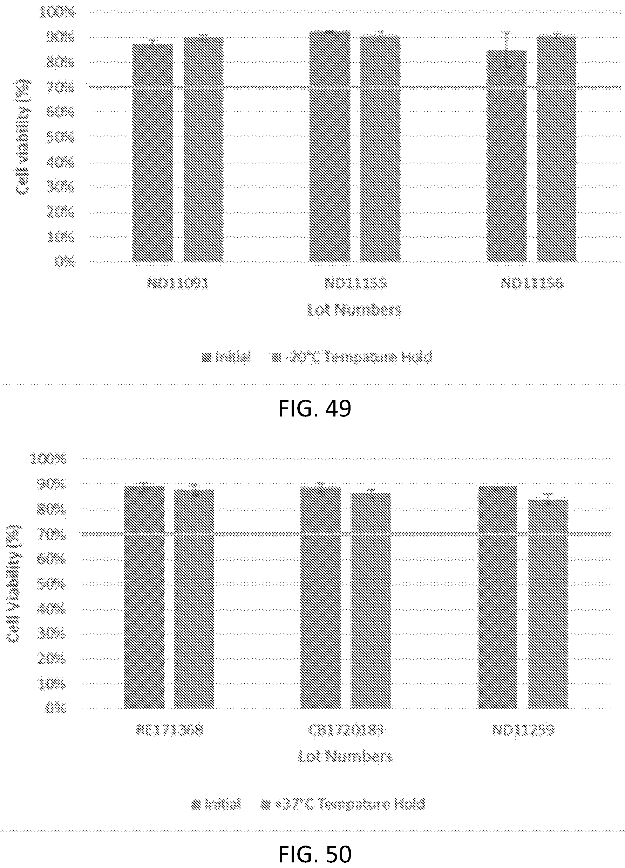

Final VLAM products can be stored and shipped at ambient temperatures, however, during shipment a broad range of temperature fluctuations can occur depending on the time of the year and the destination. The purpose of these experiments was to evaluate impact of different temperatures on VLAM for a minimum duration of 72 hours, anticipated shipment time. Cell viability test was used for evaluation of VLAM samples in this experiment. Following antibiotic treatment, AMs from three donors were prepared. VLAM samples immediately after lyophilization served as a baseline, a control. Those samples (3 units per donor) were submitted for cell viability testing when the lyophilization was completed. Other VLAM samples were exposed to -20°C, 37°C, and 50°C for a minimum of 72 hours. The exposure time was as follows: for -20°C - 94 hrs. 30 mins; for 37°C -77 hrs. 34 mins; and for 50°C is 92 hrs and 15 mins. The temperature range from -20°C to +50°C covers anticipated temperature fluctuations during VLAM shipment. Three VLAM samples per donor for each test condition were submitted to QC for cell viability testing. A mean percent of cell viability and standard deviation were calculated for each group. A T-test was performed to determine whether there are significant differences in the percent of cell viability between experimental and control groups. All tested samples met the acceptance criterion of >70% of cell viability. The average percent of cell viability for VLAM control was 88% ± 2.91%; for VLAM exposed to - 20°C, 37°C and 50°C the average % of cell viability was 90% ± 2.91%, 86% ± 1.95% and 91% ± 2.13%, respectively. There were no statistical significant differences for % of cell viability for all temperature versus the control: p=0.3294 for -20 C, p=0.1218 for 37°C and p=0.5451 for 50°C. Results (Figures 48-50) concluded that VLAM can be exposed to temperatures ranged from -20°C to +50°C for minimal 72 hours without negative impact. Therefore, VLAM samples do not require specialized containers for shipment.

### ii. Extension for Tissue Expiration Time

The purpose of this experiment was to determine whether a total manufacturing time of 12 days including 7 days elapse from tissue collection until start of the processing is acceptable. The acceptability was evaluated by testing of VLAM cell viability. Following a minimum of seven days storage of collected placentas at 4°C, AMs from three donors were undergoing aseptic processing. Bioburden samples were collected, then AMs were split into equal two parts: one part (group #1) was incubated in the antibiotic solution for 20 ± 2 hours, and the second part (group #2) was incubated in the antibiotics for 90 ± 4 hours. After incubation in antibiotics was completed, both groups were aseptically packaged and placed in a - 80°C freezer for a minimum of 6 hours prior to lyophilization. Temperature probes were used to track temperature kinetics to ensure that after 6 hours AM samples were cryopreserved. VLAM final samples were submitted for testing: EGF, cell viability and residual moisture content (conducted by WuXi). A mean and standard deviation were calculated for each test. A T-test was performed to determine whether there are significant differences between two groups. Cell viability was 87.1% and 91.6% for group 1 (20 hr in antibiotics) and 2 (90 hr in antibiotics), respectively with no significant differences between two groups (p=0.079). EGF levels were 151.1 µg/ml and 109.7 µg/ml for group 1 (20 hr in antibiotics) and 2 (90 hr in antibiotics), respectively with no significant differences between two groups (p=0.19). Mean residual moisture content was 3.33% and 3.91% for group 1 (20 hr in antibiotics) and 2 (90 hr in antibiotics), respectively with no significant differences between two groups (p=0.31). All tested samples met the acceptance criteria described in table 4 (amniotic membrane product lot release cell viability and EGF criteria) and for residual moisture. Results show that tissue processing can be started 7 days post-collection.

### iii. Evaluation of a lyophilization effect on VLAM graft sizes.

This set of experiments was addressing a question of whether lyophilization can change VLAM graft sizes. VLAM 5x5 cm, 2x3 cm, and 16 mm grafts derived from three donors were tested. The graft dimensions prior and post-lyophilization were measured twice for each graft. Graft dimensions for 5x5 cm and 16 mm disc post-lyophilization were compared to those prior to lyophilization and the differences were calculated. Results are summarized in table 13. Results demonstrate that the impact of lyophilization on graft size is negligible. Based on these results no need for adjust graft sizes prior to lyophilization to meet graft dimensions on the VLAM label.

**Table 13. AM graft sizes prior and post-lyophilization**

| **Label Unit Size** | **5 x 5 cm** | **16 mm** |
|---|---|---|
| Pre Lyo mean measurements | 5.5 x 5.1 | 16.22 |
| Post Lyo mean measurements | 5.3 x 5.0 | 16.27 |
| mean change in size | - 0.2 x 0.1 | 0.05 |
| Recommended Size | 5.3 x 5.3 | 16 |

As a result of this study, a process has been established for manufacturing of VLAM products with predefined specifications (>70% viable cells, 7.8 pg/mL EGF and <9.70% residual moisture content).

### D. Example 4

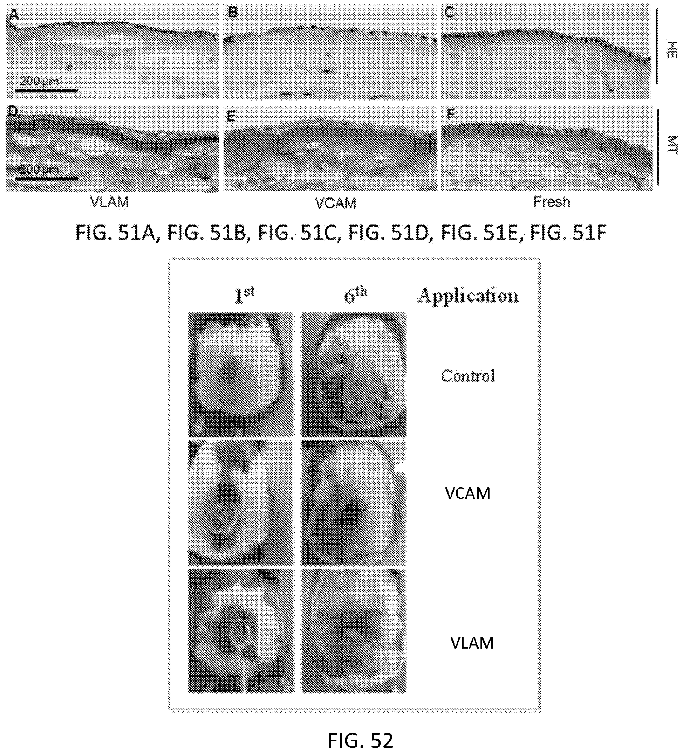

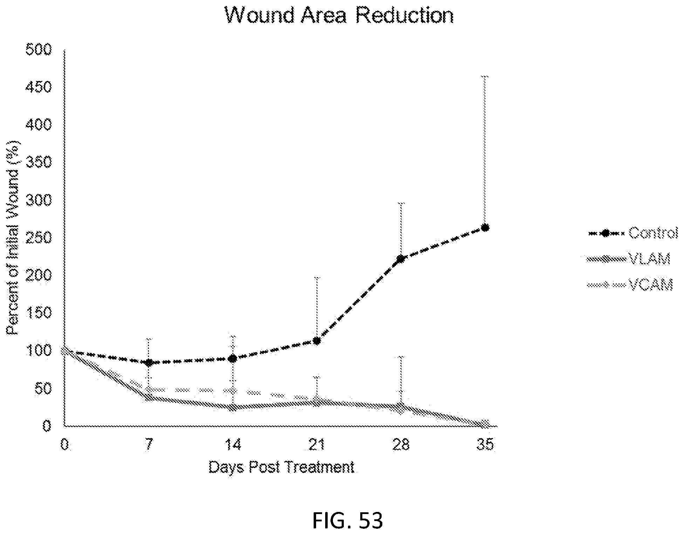

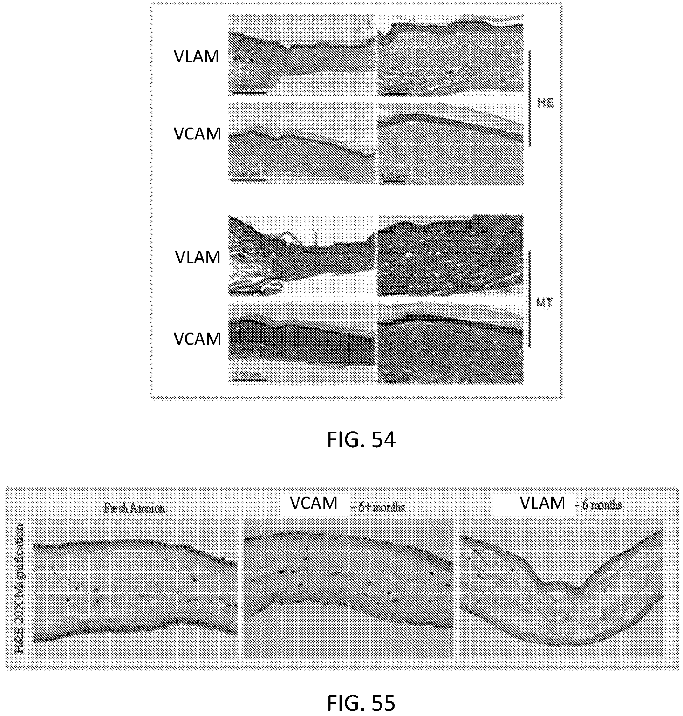

Figures 51-C shows the structural tissue integrity of fresh tissue, cryopreserved tissue and lyophilized tissue. Figures 52-54 show wound covering *in vivo* in a diabetic mouse model of chronic wound. Figure 55 shows the stability histology of fresh tissue vs lyophilized tissue.

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.
The present application also includes the subject matter of the following numbered paragraphs which corresponds to the subject matter as originally claimed in PCT Application No. PCT/US2017/039123 (WO2017/223520) from which this application is ultimately derived:
1. A method of lyophilizing a tissue sample comprising
   a) obtaining a tissue sample;
   b) contacting the tissue sample with a lyoprotectant solution
   c) freezing the tissue sample;
   d) performing a first drying step of the tissue sample after freezing; and
   e) performing a second drying step of the tissue sample after the first drying step.
2. The method of paragraph 1, wherein the tissue is placental tissue, bone, cartilage, or skin.
3. The method of paragraph 2, wherein the placental tissue is amniotic tissue, chorionic tissue, umbilical cord tissue, or a combination thereof.
4. The method of any one of paragraphs 1-2, further comprising a step of reconstituting the lyophilized tissue.
5. The method of paragraph 4, wherein the reconstituted tissue comprises at least 70% viable cells compared to the cell viability prior to lyophilization.
6. The method of any one of paragraphs 1-5, wherein the lyoprotectant solution comprises trehalose.
7. The method of any one of paragraphs 1-6, wherein the freezing is performed at a temperature range of -80°C to -4°C.
8. The method of any one of paragraphs 1-7, wherein the freezing rate is between 0.1 and 5°C/min.
9. The method of any one of paragraphs 1-7, wherein the first drying step occurs between -45°C and -15°C.
10. The method of any one of paragraphs 1-9, wherein the temperature of the first drying step is the same as the freezing temperature.
11. The method of any one of paragraphs 1-9, wherein the temperature increases from the first drying step to the second drying step.
12. The method of any one of paragraphs 1-9, wherein the second drying step is conducted at a temperature that is greater than the temperature of the freezing step.
13. The method of paragraph 11, wherein the rate of temperature increase from the first drying step to the second drying step is 0.1 to 5°C/min.
14. The method of any one of paragraphs 1-13, wherein the second drying step occurs at a temperature of no more than 45°C.
15. The method of any one of paragraphs 1-13, wherein the second drying step is conducted at at least two different temperatures.
16. The method of paragraph 15, wherein the at least two different temperatures are at least 1° to 10° different from each other.
17. The method of paragraph 16, wherein the at least two different temperatures are at least 5° different from each other.
18. The method of paragraph 15, wherein the at least two different temperatures are each maintained for 30 sec to 1 minute.
19. The method of any one of paragraphs 1-18, wherein the second drying step is conducted for 12-72 hours.
20. The method of any one of paragraphs 1-19, wherein the tissue sample is cut to a desired size prior to freezing the tissue sample, after drying the tissue sample, or after the tissue sample is reconstituted.
21. The method of any one of paragraphs 1-20, wherein the tissue sample is minced prior to freezing.
22. The method of any one of paragraphs 1-21, wherein the tissue sample is treated with an antibiotic prior to freezing.
23. The method of any one of paragraphs 1-22, wherein the lyoprotectant solution further comprises one or more antioxidants.
24. The method of paragraph 23, wherein the one or more antioxidants is catechin.
25. A lyophilized tissue prepared using the method of any of paragraphs 1-24.
26. A lyophilized tissue prepared using the method of any of paragraphs 1-24 sealed inside a sterile package.
27. The lyophilized tissue of paragraph 26, wherein the lyophilized tissue is stable for at least three months.
28. A method of treating a wound or tissue defect comprising administering a previously lyophilized tissue to the wound or tissue defect.
29. The method of paragraph 28, wherein the wound is selected from the group consisting of a laceration, a scrape, an abrasion, a thermal or chemical burn, an incision, a puncture, a wound caused by a projectile, a chronic wound, an acute wound, an external wound, an internal wound, a congenital wound, an ulcer, and combinations thereof.
30. The method of paragraph 28, wherein the wound or tissue defect is in connection with surgery.
31. The method of paragraph 30, wherein the surgery is selected from the group consisting of a tendon surgery, a ligament surgery, a bone surgery, a spine surgery, a laminectomy, a knee surgery, a shoulder surgery, a hand surgery, an elbow surgery, a toe surgery, a foot surgery, an ankle surgery, a laprascopic surgery, an endoscopic surgery, robotic surgery, an open abdominal surgery, or combinations thereof.

## Claims

1. A lyophilized bone repair composition comprising:
(a) cancellous bone particles, and
(b) periosteum,
wherein one or more angiogenic growth factors are comprised within the lyophilized composition.

2. The composition of claim 1, wherein the one or more angiogenic factors are selected from VEGF (vascular endothelial growth factor), PDGF (platelet derived growth factor), bFGF (basic fibroblast growth factor), IGF-1 (insulin-like growth factor-1), IGF-2 (insulin-like growth factor-2), TGF-β1 (transforming growth factor-beta-1), BMP-2 (bone morphogenetic protein-2) or BMP-7 (bone morphogenetic protein-7).

3. The composition of claim 1, wherein the composition further comprises a lyoprotectant.

4. The composition of claim 3, wherein the lyoprotectant comprises trehalose.

5. The composition of claim 4, wherein the trehalose is present at a concentration of 0.25M to 0.5M.

6. The composition of claim 1, wherein the composition further comprises native viable cells.

7. The composition of claim 6, wherein the native viable cells comprise osteoblasts and/or osteoclasts.

8. The composition of claim 1, wherein the composition is reconstituted.

9. The composition of claim 8, wherein the composition is reconstituted with and comprises water, saline, a buffer, an electrolyte solution, and/or human bodily fluid.

10. The composition of claim 8 for use in a method of treating a bone tissue defect, the method comprising administering the composition to the bone tissue defect.

11. The composition for use according to claim 10, wherein the tissue defect is associated with a surgery selected from a tendon surgery, a ligament surgery, a bone surgery, a spine surgery, a laminectomy, a knee surgery, a shoulder surgery, a hand surgery, an elbow surgery, a toe surgery, a foot surgery, or an ankle surgery.

12. The composition for use according to claim 10, wherein the composition is reconstituted with and comprises water, saline, a buffer, an electrolyte solution, and/or human bodily fluid.
